# EUROPEAN PATENT SPECIFICATION

(11) **EP 1 855 603 B1**
(45) Date of publication and mention of the grant of the patent: **14.10.2009**
(21) Application number: 06719745.9
(22) Date of filing: 31.01.2006
(51) Int. Cl.: A61B 10/00, A61B 17/00

(54) **END EFFECTOR FOR SURGICAL INSTRUMENT**
ENDEFFEKTOR FÜR EIN CHIRURGISCHES INSTRUMENT
ORGANE EFFECTEUR DESTINE A UN INSTRUMENT CHIRURGICAL

(30) Priority: 31.01.2005 US 648538 P; 18.11.2005 US 738279 P; 30.01.2006 US 343294
(43) Date of publication of application: 21.11.2007
(73) Proprietor: Ethicon Endo-Surgery, Inc., Cincinnati, OH 45242 (US)
(72) Inventor: BALES, Thomas, O., Coral Gables, Florida 33156 (US); PALMER, Matthew, A., Miami, Florida 33156 (US); SMITH, Kevin, W., Coral Gables, Florida 33156 (US); KLINE, Korey, Robert, Miami, Florida 33143 (US); DEVILLE, Derek, Dee, Coral Gables, FL 33156 (US)
(74) Representative: Hoffmann, Jörg Peter
(86) International application number: PCT/US2006/003029
(87) International publication number: WO 2006/083728

(56) References cited:
- DE-U1- 9 005 519
- US-A- 2 994 321
- US-A- 4 632 110
- US-A- 5 275 615
- US-A- 5 320 627
- US-A- 5 394 885
- US-A- 5 482 054
- US-A1- 2004 260 198
- US-B1- 6 238 414
- US-B1- 6 273 860
- US-B1- 6 818 007

## Description

### Technical Field

The present invention relates to an end effector for a surgical instrument.

The end effector of the present invention is for a surgical instrument and is made from two pieces. The pieces are joined to form the end effector. The pieces can be made by powder metal processes, machining, stamping, fine blanking, deep drawing, casting etc. The pieces can be joined by riveting, welding, soldering, brazing, folded tabs, pressing, etc. The pieces can be made from the same materials, or different materials.

Also disclosed is an end effector made from one piece of flat sheet material, preferably metal, and, in particular, heat treatable stainless steel. The end effector is specifically designed to take advantage of this low cost process.

Further disclosed is an end effector for a surgical instrument having teeth that are formed so that they curve toward the inside of the end effector and create an undercut along their inner surface

Finally, a self-centering spike for an end effector is disclosed for a surgical instrument. The spike has a piercing blade. As the jaws of the end effector are opened, the spike moves from a floating position to a secured centered position between the jaws of the end effector.

### Background Art

A number of different types of biopsy forceps are in common use, typically, in conjunction with endoscopic assistance. Ordinarily, these devices are of complicated construction, requiring the manufacturing and machining of precise miniaturized components, which are, therefore, generally quite expensive.

One early example of flexible forceps is shown in U.S. Pat. No, 3,895,636 to Schmidt, wherein a pair of cup shaped jaws having an annular rim mate with a hub and a sharpened trocar. The Schmidt jaws are of a nature that requires machining for the edge, each jaw being different from the other jaw.

United States Patent No. 4,887,612 to Esser et al. discloses a similar biopsy forceps that utilizes a cam linkage to effect movement of the cup-shaped jaws toward and away from one another. The Esser et al. jaws are made from stainless steel and, likewise, require expensive machining. United States Patent No. 4,763,668 to Macek et al. discloses a biopsy forceps whose cup-shaped forceps are driven by a linkage. Each pivot point in the linkage establishes a new place for stress, wear and breakage. This is similar to the linkage assembly shown in U.S. Pat. No. 4,721,116 to Schintgen et al. A needle between the forceps is retractable as the forceps close.

United States Patent No. 3,921,640 to Freeborn shows a surgical instrument manufactured from a single piece of molded plastic. The instrument can have any of various forms of jaws including a configuration of teeth for holding towels or surgical dressings.

United States Patent No. 4,200,111 to Harris describes a pair of spring-biased jaws slidably disposed within the end of a trocar. The jaws are moved inwardly and outwardly from the trocar by movement from a twisted wire.

United States Patent No. 4,669,471 to Hayashi shows a biopsy forceps device having a pair of cups attached by a pivot pin. There are several linkages between the cups and the operating wire, which are, likewise, connected by pivot pins. The pins are welded or fused to their components by laser welding.

United States Patent No. 4,815,460 to Porat et al. describes a medical device for gripping. The Porat et al. device has a pair of jaws identical to one another. The jaws have an array of teeth disposed completely thereacross. The teeth are divided longitudinally across each jaw and are out of phase from one another. A further device is shown in U.S. Patent No. 825,829 to Heath. The Heath appliance utilizes two different sets of engaging jaws to accomplish its cutting purpose.

United States Patent Nos. 5,507,296, 5,666,965, 6,024,707, 6,264,617 to Bales et al. describe a biological forceps device for the taking of tissue samples from a body. The forceps device has a flexible main coil and, at a distal end thereof, a pair of homologous cast jaws. The jaws have radially disposed teeth on their distalmost end. The jaws are opened and closed by attachment to a pair of pull wires that extend through the main coil, into a handle at a proximal end of the coil. The handle has a spool that slides about a central shaft attached to the main coil. The spool is attached to the pull wires so that movement of the spool with respect to the central shaft effectuates a force on the proximal ends of the levered jaws to open and close them.

What is needed is an improved jaw assembly that grasps tissue better, travels inside a working channel of an endoscope (or other scope), and is easier to manufacture with decreased cost.

In flexible endoscopy, long, flexible instruments are inserted into and withdrawn from the working channel of a flexible endoscope frequently during a given surgical procedure. The endoscopist does this quickly to help move the procedure along. Sometimes, upon rapid withdrawal, the instrument accidentally exits the scope and whips into the air, flinging body fluids around the room and potentially in the endoscopist's or nurse's face. To help prevent this undesirable situation, indicator marks have been added to the distal portion of the sheath to give the physician a visual cue that the tip of the instrument is approaching. The outer sheath is, typically, an opaque color, the color indicating the length of the device. Marks are also added to the proximal portion of the sheath to indicate, upon insertion, that the instrument is almost deep enough to exit the distal end of the endoscope. These marks are printed on the outside of the polymer sheath. Currently, such printing is performed using hot stamping or pad printing. The polymer outer sheath is delicate and can be damaged during printing. It would be desirable to eliminate any possibility of damaging the outer sheath by adding such markers.

Flexible endoscopes are expensive reusable devices, and the cost of repairing a worn out working channel is considerable. Because biopsy forceps are passed so frequently through the working channel of a flexible endoscope, the channel is subject to wear. It is, therefore, advantageous to design the end effector of the biopsy forceps such that it minimizes scope channel wear.

Document US 2004/0260198 A1 discloses endoscopic instruments comprising an end effector assembly which has an end effector with non-sharp edges and corners for instance in the form of a biopsy jaw with a tissue receiving portion. Provided opposing jaw portions have a plurality of teeth, wherein each of the teeth includes a crest, a root and an intermediate portion between the crest and the root and wherein intermediate portions of opposing jaws are configured in order to contact each other when the opposing jaw portions are brought together.

Document DE 90 05 519 U1 discloses biopsy forceps comprising a shaft having mounted thereon a fixed jaw portion. The fixed jaw portion has provided thereon a pivotally supported second jaw portion. The fixed jaw portion is disposed in the plane of a closing movement in an angle with respect to the axis of the shaft.

### Disclosure of Invention

It is accordingly an object of the present invention to provide an end effector for a surgical instrument that overcomes the hereinafore-mentioned disadvantages of the heretofore-known devices and methods of this general type.

The objects underlying the present invention are achieved by an end effector according to independent claim 1. Preferred embodiment of the end effector are defined in the dependent claims.

The forceps teeth are recurved to curve inwards and create an undercut on their inner surface. The undercut traps the tissue that is being grasped and holds it better (like a hook). The angle formed by the recurve cams the teeth deeper into the tissue as the end effectors are being closed.

The tang of the end effector of the present invention can be used for many different style end effectors.

It is beneficial if the end effector is formed from two pieces that are connected together. Therefore, the tang and the end effector can be made by different processes to allow for flexibility in constructing each piece of the end effector and to take advantage of differing manufacturing processes.

Alternatively, the end effector can be made from a single sheet of thin material configured for inherent strength. Preferably, the material is a heat treatable stainless steel that can be easily formed in its annealed condition and heat treated after forming to improve its strength and hardness. In the one-piece embodiment of the jaw, the tang is formed from two separate tangs that both include axle and actuation holes.

Even though the end effector is shown as a biopsy forceps jaw in the drawings, the same tang configuration could be used for many different kinds of end effectors such as: graspers, dissectors, clamps, etc.

With the foregoing and other objects in view, there is provided, in accordance with the invention, a n end effector jaw for an endoscopic surgical instrument, including a tang portion, a hollow body portion having a lateral side with substantially straight opposing edges and a proximal portion connecting the lateral side to the tang portion, and a hollow nose portion connected to the lateral side and having substantially linear edges at an angle to the edges of the lateral side. The hollow body and nose portions define a biopsy cup for receiving a tissue sample therein and the opposing and linear edges form a pinching surface for contacting extremities of the tissue sample.

In accordance with another feature of the invention, the edges of the nose portion are each connected at an angle to a respective one of the opposing edges of the lateral side.

In accordance with a further feature of the invention, the linear edges of the nose portion are two linear edges, the opposing edges of the lateral side are two opposing edges, and the two edges of the nose portion are each connected at an angle to a respective one of the two opposing edges.

In accordance with an added feature of the invention, the linear edges of the nose portion are two linear edges.

In accordance with an additional feature of the invention, the two linear edges oppose one another, in particular, the two linear edges oppose one another at an angle.

In accordance with yet another feature of the invention, the nose portion forms one half of a frustoconical body having two substantially linear side edges, a substantially linear front edge, and a substantially linear front face.

In accordance with yet a further feature of the invention, the nose portion has a side portion connected to the lateral side and having the linear edges at the angle to the lateral side and a front portion having a substantially linear front face and a linear edge connected at an angle to each of the edges of the side portion.

In accordance with yet an added feature of the invention, the side portion and the front portion form one half of a frustoconical body.

In accordance with yet an additional feature of the invention, the side portion is a set of angled side portions.

In accordance with again another feature of the invention, the set of angled side portions forms one half of a bi-frustoconical body.

In accordance with again a further feature of the invention, at least one of the opposing edges and the linear edges has at least one tooth. In one embodiment, the opposing edges and the linear edges each have teeth.

In accordance with again an added feature of the invention, the linear edge of the front portion has at least one tooth.

In accordance with again an additional feature of the invention, the tang portion, the body portion, and the nose portion are integral.

In accordance with still another feature of the invention, the tang portion, the body portion, and the nose portion are stamped from a single piece of material.

With the objects of the invention in view, there is also provided an end effector for an endoscopic surgical instrument having a longitudinal body with proximal and distal ends and an actuator at the proximal end of the body, the end effector including a clevis to be connected to the distal end of the body and two jaws, each of the jaws having a tang portion pivotally connected to the clevis and to be connected to the actuator for pivoting the jaw, a hollow body portion having a lateral side with substantially straight opposing edges and a proximal portion connecting the lateral side to the tang portion, and a hollow nose portion connected to the lateral side and having substantially linear edges at an angle to the edges of the lateral side. The hollow body and nose portions define a biopsy cup for receiving a tissue sample therein and the opposing and linear edges form a pinching surface for contacting extremities of the tissue sample.

In accordance with still a further feature of the invention, for each of the jaws, the edges of the nose portion are each connected at an angle to a respective one of the opposing edges of the lateral side.

In accordance with still an added feature of the invention, the jaws have a closed orientation and the nose portion of each of the jaws forms one half of a frustoconical body that is fully formed by both of the jaws when the jaws are in the closed orientation.

In accordance with still an additional feature of the invention, each of the opposing edges of the lateral side and the linear edges of the nose portion define an abutting periphery for each of the jaws, a junction of the opposing edges of the lateral side and the linear edges of the side portion at the abutting periphery of each of the jaws defines a first intersection, and the abutting periphery has areas containing teeth and tooth-free areas at the first intersection.

In accordance with another feature of the invention, the nose portion has a front face with a front edge and a proximal portion having the linear edges, a junction of the front edge and the linear edges at the abutting periphery of each of the jaws defines a second intersection, and the abutting periphery contains tooth-free areas at the first and second intersections.

In accordance with a further feature of the invention, the the front face is substantially planar and the front edge is substantially linear and has at least one tooth.

In accordance with an added feature of the invention, the end effector is to be inserted into a working channel of an endoscope and contact between the end effector and the working channel is to occur substantially at the first and second intersections.

With the objects of the invention in view, there is also provided an endoscopic surgical instrument, including a hollow body having a distal end and a proximal end, the end effector of the present invention, the clevis connected to the distal end of the body to attach the end effector to the distal end of the hollow body, and an actuator disposed at the proximal end of the hollow body and connected through the hollow body to the tang portion of at least one of the jaws to pivot at least one of the jaws relative to the other of the jaws when actuated. The actuator pivots the at least one jaw to engage the opposing edges of the lateral side and the linear edges of the nose portion of one of the jaws with the opposing edges and the linear edges of the other of the jaws when actuated.

In accordance with an additional feature of the invention, the jaws have a closed orientation, and the nose portion of each of the jaws forms one half of a frustoconical body that is fully formed by both of the jaws when the jaws are in the closed orientation.

With the objects of the invention in view, there is also provided an endoscopic surgical instrument, including a hollow body having a distal end and a proximal end, an end effector having a clevis connected to the distal end of the body and two jaws. Each of the jaws have a tang portion pivotally connected to the clevis, a hollow body portion having a lateral side with substantially linear opposing edges, and a proximate portion connecting the lateral side to the tang portion, and a hollow nose portion connected to the lateral side and having substantially linear edges at an angle to the edges of the lateral side. The hollow body and nose portions define a biopsy cup for receiving a tissue sample therein and the opposing and linear edges form a pinching surface for contacting extremities of the tissue sample. An actuator is disposed at the proximal end of the body and connected through the body to the tang portion of the jaws to pivot at least one of the jaws relative to the other of the jaws when actuated.

In accordance with yet another feature of the invention, for each of the jaws, the edges of the nose portion are each connected at an angle to a respective one of the opposing edges of the lateral side.

In accordance with yet a further feature of the invention, each of the opposing edges of the lateral side and the linear edges of the nose portion define an abutting periphery for each jaw, a junction of the opposing edges of the lateral side and the linear edges of the side portion at the abutting periphery of each of the jaws defines a first intersection, and the abutting periphery has areas containing teeth and tooth-free areas at the first intersection.

In accordance with yet an added feature of the invention, the nose portion has a front face with a front edge and a proximal portion having the linear edges, a junction of the front edge and the linear edges at the abutting periphery of each of the jaws defines a second intersection, and the abutting periphery contains tooth-free areas at the first and second intersections.

In accordance with yet an additional feature of the invention, the front face is substantially planar and the front edge is substantially linear and has at least one tooth.

In accordance with again another feature of the invention, the end effector is to be inserted into a working channel of an endoscope and contact between the end effector and the working channel is to occur substantially at the first and second intersections.

With the objects of the invention in view, there is also provided an end effector jaw for an endoscopic surgical instrument, including a hollow body portion having a proximal tang and a distal body with lateral sides and a hollow nose portion connected to the body portion and having substantially linear edges at an angle to the lateral side. The hollow body and nose portions define a biopsy cup for receiving a tissue sample therein and the opposing and linear edges form a pinching surface for contacting extremities of the tissue sample.

In accordance with again a further feature of the invention, the nose portion has a proximal portion connected to the body portion and having the substantially linear edges at an angle to the lateral side and a front portion having a substantially linear front face with a substantially linear edge connected to the linear edges of the proximal portion.

With the objects of the invention in view, there is also provided an end effector jaw for an endoscopic surgical instrument, including a body portion having a proximal tang and a distal body with lateral sides and a half-frustoconical nose portion connected to the distal body.

With the objects of the invention in view, there is also provided an end effector for an endoscopic surgical instrument having a longitudinal body with proximal and distal ends and an actuator at the proximal end of the body, the end effector including a clevis to be connected to the distal end of the body and two jaws, at least one of the two jaws being pivotally connected to the clevis, each of the jaws having a body portion having a proximal tang and a distal body with lateral sides, and a half-frustoconical nose portion connected to the distal body.

In accordance with again an added feature of the invention, the half-frustoconical nose portion has two substantially linear edges and a substantially linear front face having a substantially linear edge connected at an angle to the two linear edges.

With the objects of the invention in view, there is also provided an end effector jaw assembly for a clevis of an endoscopic surgical instrument, including two opposing jaws to be connected to the clevis, the jaws having a closed orientation, each of the jaws having a body portion having a proximal tang and a distal body with lateral sides, and a half-frustoconical nose portion connected to the distal body and having at least two substantially linear edge segments.

In accordance with again an additional feature of the invention, the jaws define a central longitudinal axis and each of the at least two edge segments has at least one tooth at least partially curved about the longitudinal axis.

In accordance with still another feature of the invention, the at least two substantially linear edge segments of different ones of the jaws are appositioned and the at least one tooth of each of the jaws is interdigitated when the two jaws are in the closed orientation.

With the objects of the invention in view, there is also provided an end effector jaw for an endoscopic surgical instrument, including a tang portion, a hollow body portion having a lateral side with substantially linear opposing edges and a proximal portion connecting the lateral side to the tang portion, and a hollow nose portion having a side portion having substantially linear edges connected at an angle to the opposing edges of the lateral side and a front portion having a substantially linear front face with a substantially linear edge connected at an angle to the linear edges of the side portion. The hollow body and nose portions define a biopsy cup for receiving a tissue sample therein and the opposing and linear edges form a pinching surface for contacting extremities of the tissue sample.

Other features that are considered as characteristic for the invention are set forth in the appended claims.

Although the invention is illustrated and described herein as embodied in an end effector for a surgical instrument, a surgical instrument having the end effector, and a method for forming the end effector, it is, nevertheless, not intended to be limited to the details shown because various modifications and structural changes may be made therein within the scope and range of equivalents of the claims.

The construction and method of operation of the invention, however, together with additional objects and advantages thereof, will be best understood from the following description of specific embodiments when read in connection with the accompanying drawings.

### Brief Description of Drawings

FIG. 1 is a fragmentary, perspective view of a distal end of an embodiment of a surgical instrument not according to the invention;
FIG. 2 is an exploded, perspective view of a first embodiment of a jaw assembly of the instrument of FIG. 1;
FIG. 3 is a perspective view of the jaw assembly of FIG. 2 in a connected position;
FIG. 4 is an exploded, perspective view of another embodiment of the jaw assembly of the instrument of FIG. 1;
FIG. 5 is a perspective view of the jaw assembly of FIG. 4 in a connected position;
FIG. 6 is a fragmentary, perspective view of a distal end of an end effector of the surgical instrument not according to the invention with a second embodiment of a jaw assembly with the jaws in an opened position;
FIG. 7 is a fragmentary, perspective view of the surgical instrument of FIG. 6 with the jaws in a closed position;
FIG. 8 is a fragmentary, side elevational view of the surgical instrument of FIG. 6;
FIG. 9 is a fragmentary, side elevational view of the surgical instrument of FIG. 7;
FIG. 10 is a perspective view from a right side of one jaw of the instrument of FIG. 6;
FIG. 11 is a perspective view from a left side of the jaw of FIG. 10;
FIG. 12 is an elevational view from the left side of the jaw of FIG. 10;
FIG. 13 is an elevational view from the right side of the jaw of FIG. 10;
FIG. 14 is an enlarged, fragmentary, cross-sectional view of the distal portion of the jaw of FIG. 10 viewed from the right side;
FIG. 15 is a plan view of the jaw of FIG. 10;
FIG. 16 is an elevational view from the distal end of the jaw of FIG. 10;
FIG. 17 is an enlarged, fragmentary, elevational view of the jaw of FIG. 9 from the left side of the jaw;
FIG. 18 is an enlarged, elevational view from the distal end of the jaw of FIG..9;
FIG. 19 is a perspective view of a clevis of the instrument of FIGS. 1, 6, 7, 8, and 9;
FIG. 20 is a side elevational view of the clevis of FIG. 19;
FIG. 21 is a plan view of the clevis of FIG. 19;
FIG. 22 is a plan view of the clevis,of FIG. 19 after being stamped but before being shaped;
FIG. 23 is an enlarged, perspective view of a blade according to the invention;
FIG. 24 is a perspective view of a third embodiment of a jaw assembly not according to the invention with the blade of FIG. 23 viewed from a distal end of the jaw assembly;
FIG. 25 is a perspective view the jaw assembly of FIG. 24 from a right-side thereof;
FIG. 26 is a fragmentary, enlarged cross-sectional view of a central portion of a pair of an alternative embodiment of the jaws of FIGS 10 to 18 mounted upon an axle;
FIG. 27 is a fragmentary, perspective view of a distal end of an embodiment of the end effector according to the invention viewed from distal end thereof with the jaws in an open position;
FIG. 28 is a fragmentary, perspective view of the end effector of FIG. 27 rotated approximately 25 degrees with the jaws in a closed position;
FIG. 29 is a fragmentary, perspective view of the end effector of FIG. 27 viewed from a proximal side thereof and rotated approximately 90 degrees;
FIG. 30 is a fragmentary, plan view of the end effector of FIG. 28 without the control rods;
FIG. 31 is a fragmentary, side elevational view of the end effector of FIG. 30;
FIG. 32 is a fragmentary, cross-sectional view of the end effector of FIG. 31 with a spike in a centered position;
FIG. 33 is a fragmentary, cross-sectional view of the end effector of FIG. 31 with the spike in a non-centered position
FIG. 34 is a fragmentary, cross-sectional view of the end effector of FIG..31 with the jaws in an open position and the spike in the centered position;
FIG. 35 is a fragmentary, side elevational view of the end effector of FIG. 34;
FIG. 36 is a fragmentary, perspective view of the end effector of FIG. 30;
FIG. 37 is a fragmentary, perspective view of the end effector of FIG. 30 with the jaws in the opened position;
FIG. 38 is a fragmentary, exploded, perspective view of the end effector of FIG. 30;
FIG. 39 is a fragmentary, perspective view of a jaw of the end effector of FIG. 27 viewed from distal of a distal end thereof;
FIG. 40 is a side elevational view of the jaw of FIG. 39 viewed from a right side thereof;
FIG. 41 is a side elevational view of the jaw of FIG. 39 viewed from a left side thereof;
FIG. 42 is a plan view of the jaw of FIG. 39;
FIG. 43 is a perspective view of the jaw of FIG. 27 axially rotated approximately 180 degrees;
FIG. 44 is a perspective view of the jaw of FIG. 43 centrally rotated approximately 180 degrees;
FIG. 45 is a plan view of the bottom of the jaw of FIG. 39;
FIG. 46 is a fragmentary, perspective view of the end effector of FIG. 27 viewed from distal of a distal end thereof and off-axis thereof with shading;
FIG. 47 is a fragmentary, enlarged plan view of the end effector of FIG. 30;
FIG. 48 is a fragmentary, enlarged plan view of the end effector of FIG. 30 with shading;
FIG. 49 is a fragmentary, enlarged front elevational view of the end effector of FIG. 27 viewed along the longitudinal axis thereof;
FIG. 50 is a fragmentary, enlarged front elevational view of the end effector of FIG. 27 viewed along the longitudinal axis thereof and with shading;
FIG. 51 is a fragmentary, perspective view of another embodiment of an end effector according to the invention viewed from distal of a distal end thereof and having a bi-frusto-conical distal end;
FIG. 52 is a perspective view of an alternative embodiment of a jaw of the end effector according to the invention with a single tang;
FIG. 53 is an alternative perspective view of the jaw of FIG 52;
FIG. 54 is a side elevational view of the jaw of FIG. 52;
FIG. 55 is a plan view of the jaw of FIG. 52 viewed from an interior side of the jaw;
FIG. 56 is a cross-sectional view of the jaw of FIG. 52;
FIG. 57 is an alternative cross-sectional view of the jaw of FIG. 52;
FIG. 58 is a side elevational view of a sixth embodiment of the end effector according to the invention with a pair of the jaws of FIG. 52;
FIG. 59 is a plan view of the end effector of FIG. 58 viewed from an exterior side of one of the jaws;
FIG. 60 is an enlarged, fragmentary, perspective view of a tang portion of the end effector of FIG. 58;
FIG. 61 is an alternative, enlarged, fragmentary, perspective view of the tang portion of FIG. 60;
FIG. 62 is a perspective view of the end effector of FIG. 58 viewed from the distal end thereof with the jaws in an opened position;
FIG. 63 is an alternative, perspective view of the end effector of FIG. 62;
FIG. 64 is a perspective view of the end effector of FIG. 58 viewed from a side of the proximal end thereof with the jaws in an opened position;
FIG. 65 is an enlarged perspective view of a further embodiment of the jaw assembly not according to the invention;
FIG. 66 is an enlarged, exploded perspective view of the jaw assembly of FIG. 65;
FIG. 67 is a perspective view of an alternative embodiment of a jaw of the end effector of FIG. 27;
FIG. 68 is a perspective view of an enlarged portion of the end effector of FIG. 28 having a pair of the jaws shown in FIG. 67 and rotated with respect thereto;
FIG. 69 is a perspective view of an enlarged portion of the end effector of FIG. 27 having a pair of the jaws shown in FIG. 67;
FIG. 70 is a perspective view of the jaw of FIG. 67 rotated approximately 180 degrees about its longitudinal axis;
FIG. 71 is a fragmentary, partially cross-sectional and partially hidden view of an end effector according to FIGS. 27 et seq. about to traverse a curve of a working channel of a flexible endoscope before rotating therein;
FIG. 72 is a fragmentary, partially cross-sectional and partially hidden view of the end effector of FIG. 71 traversing the curve and before rotating therein;
FIG. 73 is a fragmentary, partially cross-sectional and partially hidden view of the end effector of FIG. 71 further traversing the curve and after rotating therein;
FIG. 74 is a plan view of an alternative embodiment of the jaw of FIG. 67 viewed from a bottom thereof and illustrating a second embodiment of the jaw control wire;
FIG. 75 is a perspective view of the jaw of FIG. 74;
FIG. 76 is an enlarged perspective view of a portion of the jaw of FIG. 75;
FIG. 77 is a plan view of the jaw of FIG. 74 illustrating a third embodiment of the jaw control wire;
FIG. 78 is a perspective view of the jaw of FIG. 77;
FIG. 79 is an enlarged perspective view of a portion of the jaw of FIG. 78;
FIG. 80 is a fragmentary, side elevational view of the end effector of FIG. 34 approaching a tissue surface at an angle;
FIG. 81 is a fragmentary, side elevational view of the end effector of FIG. 80 after the end effector has been pressed against the tissue surface to rotate and align with the tissue surface;
FIG. 82 is an exploded view of an exemplary embodiment of a handle for the forceps medical instrument according to the invention;
FIG. 83 is a fragmentary, enlarged, cross-sectional view of a distal portion of the handle of FIG. 82 and an exploded view of the distal portion, a shaft retainer, and a shaft;
FIG. 84 is a fragmentary, further enlarged, cross-sectional view of the distal portion of the handle of FIG. 82 and an exploded view of the distal portion, the shaft retainer, and the shaft;
FIG. 85 is a fragmentary, cross-sectional view of the distal portion of the handle, the shaft retainer, and the shaft of FIG. 84;
FIG. 86 is an enlarged, perspective view of a first exemplary embodiment of the shaft retainer of FIGS. 82 to 85;

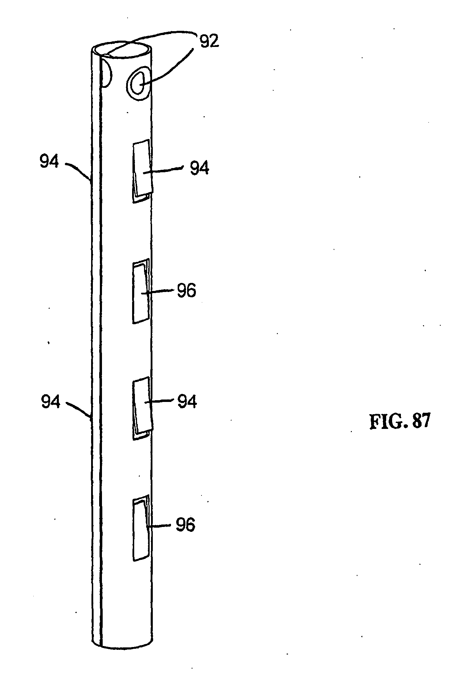

FIG. 87 is an enlarged, perspective view of a second exemplary embodiment of the shaft retainer of FIGS. 82 to 85;
FIG. 88 is a side elevational view of the handle of FIG. 82 and an exploded view of the handle and the shaft assembly according to the invention, including an actuation rod, the shaft retainer, and the shaft;
FIG. 89 is a diagrammatic illustration of the medical instrument according to the invention with a forceps, a handle, and an exemplary embodiment of markers; and
FIG. 90 is a fragmentary, enlarged, broken away side elevational view of the shaft of the instrument of FIG. 82 with markers on a coil of the shaft.

### Best Mode for Carrying Out the Invention

While the specification concludes with claims defining the features of the invention that are regarded as novel, it is believed that the invention will be better understood from a consideration of the following description in conjunction with the drawing figures, in which like reference numerals are carried forward.

Before the present invention is disclosed and described, it is to be understood that the terminology used herein is for the purpose of describing particular embodiments only and is not intended to be limiting. It must be noted that, as used in the specification and the appended claims, the singular forms "a," "an," and "the" include plural references unless the context clearly dictates otherwise.

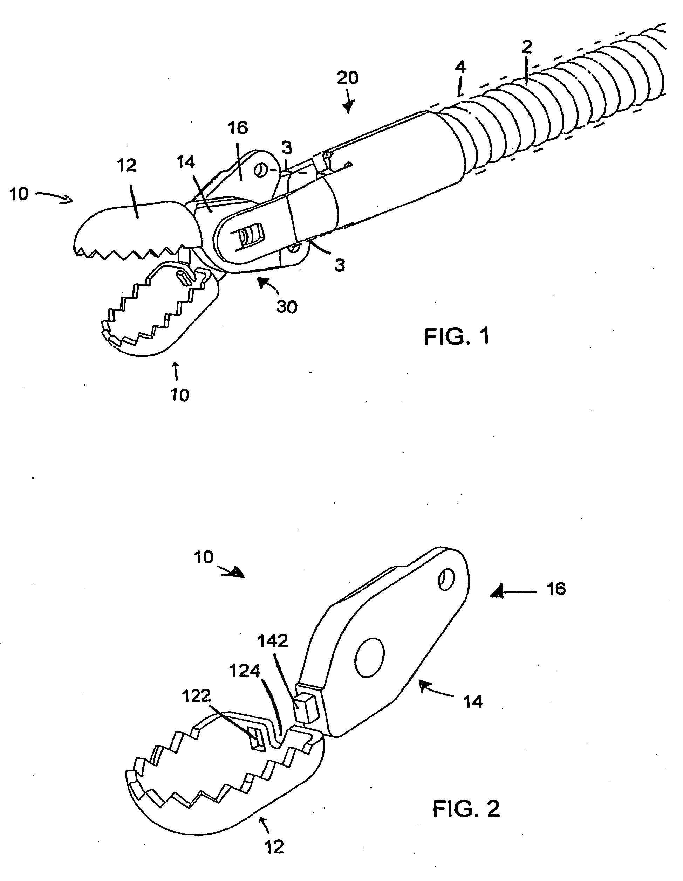

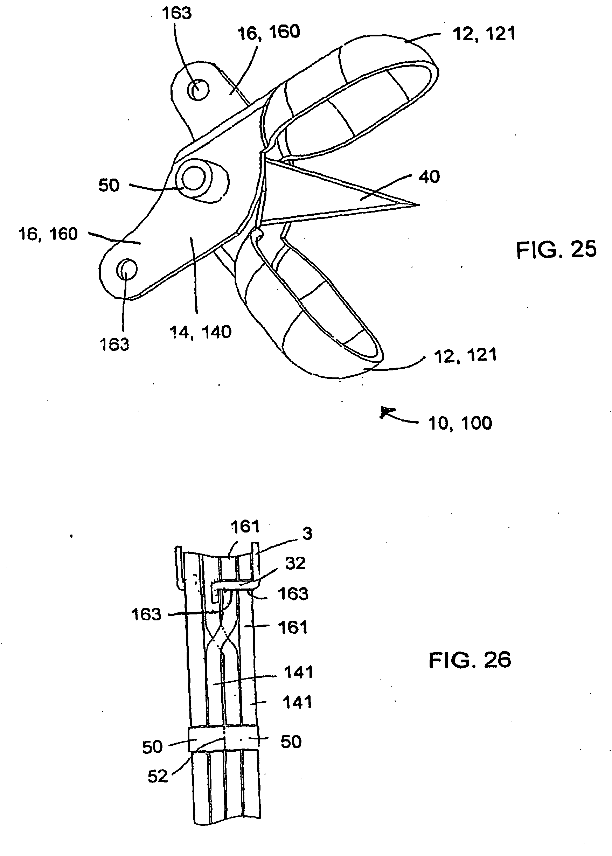

Referring now to the figures of the drawings in detail and first, particularly to FIG. 1 thereof, there is shown a diagrammatic illustration of an embodiment of a end effector 1 for a surgical instrument not according to the invention, in particular, the end effector 1 is a jaw actuation assembly for a biopsy forceps. The end effector includes jaws 10, a clevis 20, and a mounting axis 30. The surgical instrument includes a flexible coil 2 in which actuating wires or rods 3 (described in further detail below with regard to FIGS. 26 to 29 and only illustrated in FIG. 1 diagrammatically with dashed lines) are disposed slidably. While a covering for the coil 2 is not necessary, in a preferred embodiment, a protective material 4 can cover the coil 2. The material 4 is in the form of a watertight, shrink-sealed tubing, shown only diagrammatically in FIG. 1.

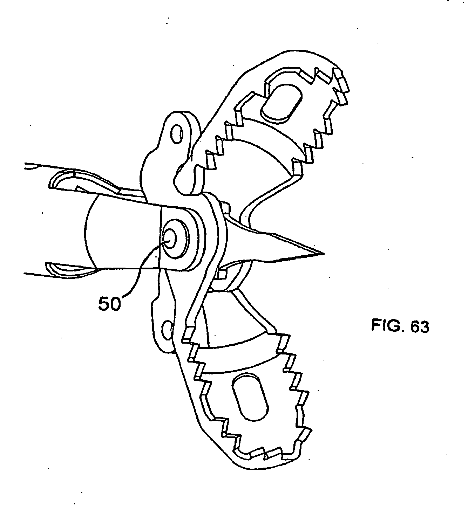

A proximal end of the actuating wires 3 is connected to a proximal actuator 60 (see FIGS. 63 to 70), which is, typically, in the form of a plunger that moves the wires 3 relative to the coil 2. A movement in a first direction closes the jaws 10 and a movement in a second direction opens the jaws. If desired, the actuator can include a locking mechanism that secures the jaws 10 from opening again once the locking mechanism is locked. Thus, in a biopsy forceps configuration, after the tissue sample is obtained inside the jaws 10, the lock will prevent the jaws from opening and, therefore, drop the tissue sample. Further description of the actuator 60 is set forth below with respect to FIGS. 63 to 70.

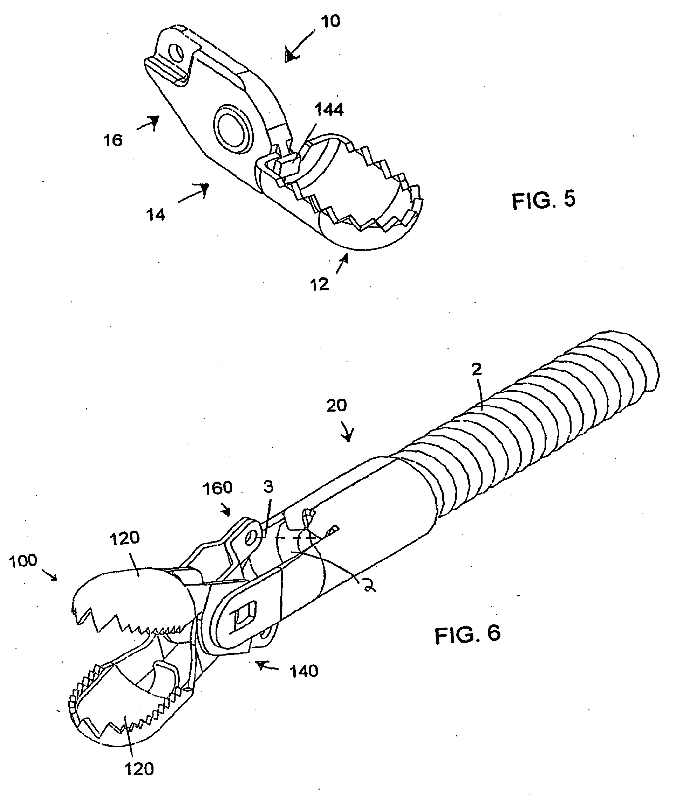

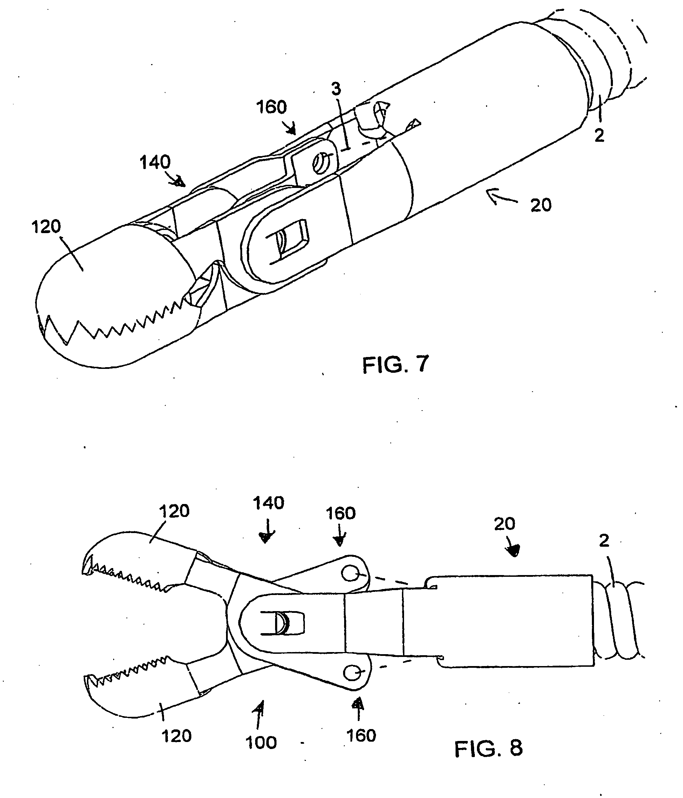

The jaws 10 include a distal mouth 12, a central mounting frame 14, and a proximal tang 16. The mouth 12, in a biopsy forceps configuration, is used to obtain the tissue specimen. The mounting frame 14 pivotally connects the jaw 10 to the clevis 20 so that the jaw 10 can be pivoted between the open and closed positions. See FIGS. 6 and 7, respectively. The tang 16 has a connection area, preferably, in the form of a bore for receiving a wire or rod. Accordingly, to connect the actuation assembly to the jaws 10, the distal end of each of the wires 3 is connected to the tang 16 of a respective one of the jaws 10. Thus, when actuated, the wires 3 move proximally or distally to pivot both jaws 10 and, thereby, close or open the jaws 10.

FIGS. 2 to 5 illustrate an embodiment of a two-piece jaw 10. The distal mouth 12 forms a first of the two pieces of the jaw 10 and the frame 14 and tang 16 form the second of the two pieces of the jaw 10. The two pieces can be connected in various ways.

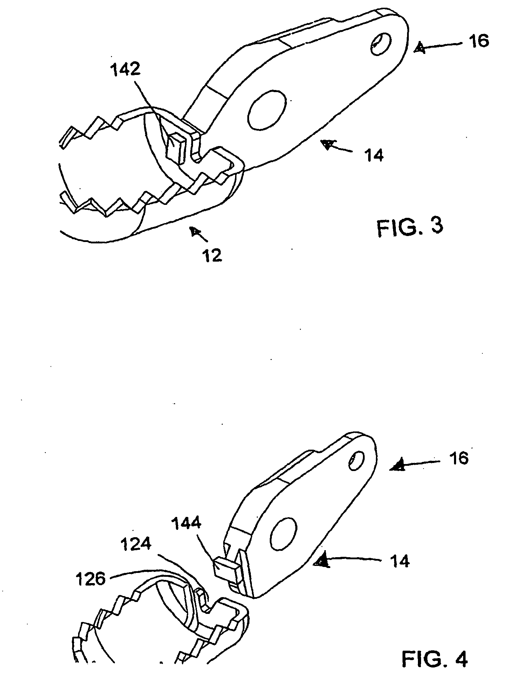

FIGS. 2 and 3 illustrate a first connection assembly. The proximal face of the mouth 12 has two features, a connection slot 122 and a groove 124 to provide clearance for a central anchoring spike or needle. The distal face of the frame 14 has a tab 142 projecting in a distal direction. The shape of the slot 122 substantially corresponds to the outer shape of the tab 142. FIG. 2 illustrates the two pieces separated from one another and FIG. 3 illustrates the tab 142 inside the slot 122. A preferred method of connecting the two pieces is to make the longitudinal extent of the tab 142 be longer than the depth of the slot 122 (which is defined, primarily, by the thickness of the material making up the mouth 12). Such a configuration is shown in FIG. 3. After the tab 142 is in this position, the two pieces are held together and the distal end of the tab 142 is crushed (in the manner of a rivet) to connect permanently the two pieces together.

FIGS. 4 and 5 illustrate a second connection assembly of the jaw 10. The proximal face of the mouth 12 has two features, a connection groove 126 and a groove 124 to provide clearance for a central anchoring spike or needle. The distal face of the frame 14 has a T-shaped tab 144 having a base projecting in a distal direction and a top projecting on either side of the base in a direction orthogonal to the base. The shape of the connection groove 126 substantially corresponds to the outer shape of the base of the tab 144. FIG. 4 illustrates the two pieces separated from one another and FIG. 5 illustrates the tab 144 inside the connection groove 126. Connecting the two pieces together simply requires sliding the base of the tab 144 through the connection groove 126 to the bottom thereof. If the longitudinal extent of the base of the tab 144 substantially corresponds to the depth of the groove 126 (corresponding to the thickness of the material making up the mouth 12), then the T-shape of the tab 144 is enough to connect the two pieces together. For additional connection strength, the top of the T-shape can be pressed and deformed (in the manner of a rivet) to connect permanently the two pieces together. Alternatively, they can be welded, brazed, or soldered together, for example.

Preferably, the two pieces are formed by stamping in one step. All of the features shown in FIGS. 2 to 5 can be formed by the single-step stamping of each of the two pieces.

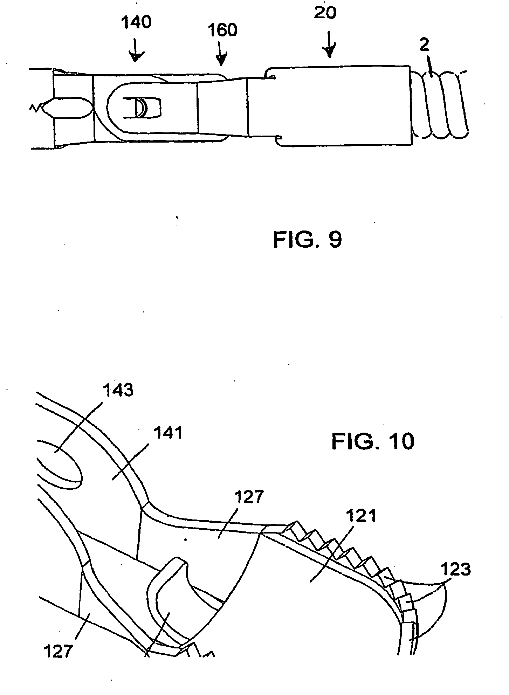

FIGS. 6 to 18 illustrate another embodiment of the jaw 100 not according to the present invention. The jaw 100 is connected pivotally to the clevis 20, which is, in turn, connected to the coil 2. The jaw 100 of FIGS. 6 to 8 is not in two pieces. Instead, this jaw 100 is a one-piece part and is formed by stamping a sheet of material into a flat stamped part and, then, shaping the stamped part into the form shown, in particular, in FIGS. 10 to 18. Thus, the jaw 100 is formed in two-steps: stamping a flat sheet of the material and shaping the stamped part. If desired, a third step can be added to connect permanently the two proximal ends of the tang 160 to one another, for example, by welding, brazing, interlocking a tab in a groove, or any other similar process.

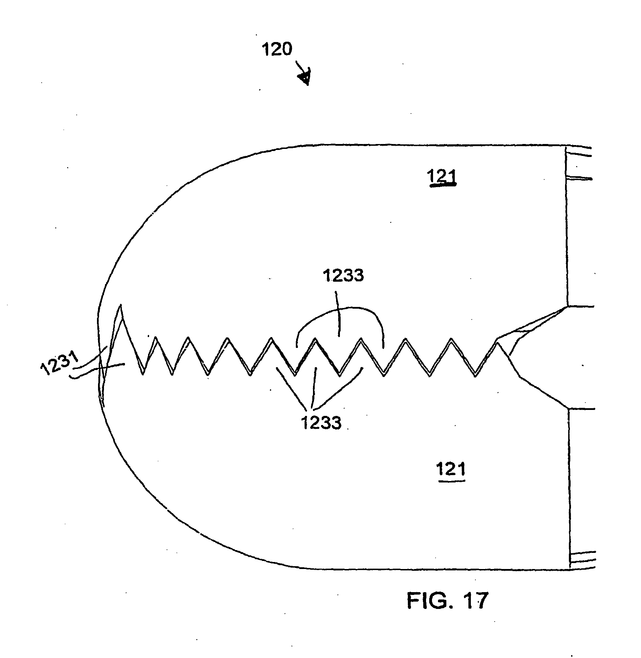

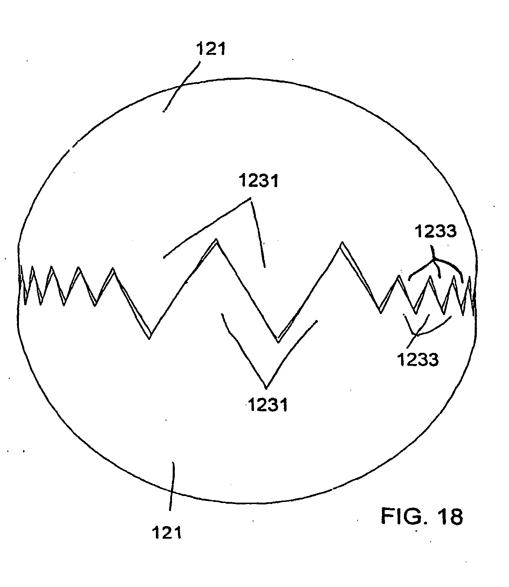

FIG. 10 clearly shows the features of the one-piece jaw 100. The mouth 120 has a cup-shaped distal portion 121 having radial teeth 123, a proximal tab 125, and two proximal arms 127. The radial teeth 124 project in the direction of the other jaw 100 and are formed to intermesh with teeth in the other jaw 100 as shown, in particular, in FIGS. 17 and 18. The proximal tab 125 is used to retain the tissue sample without limiting the cup volume. The two proximal arms 127 are connected to the distal end of the frame 14 and are integral with the frame 14.

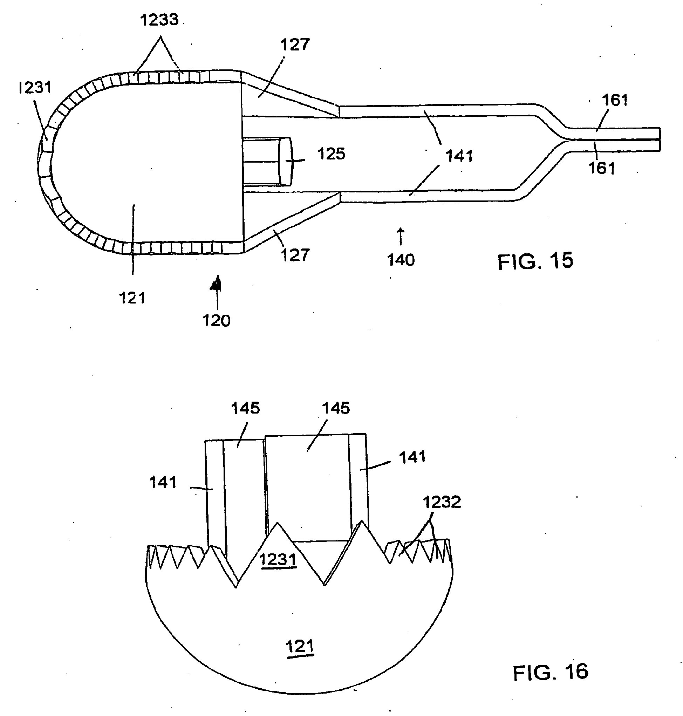

The frame 14 is made of two substantially parallel plates 141 each defining a pivot port 143. The frame 14 is connected at its distal end to the proximal arms 127 and at its proximal end to the tang 16. Like the frame 14, the tang 16 is formed from two parallel plates 161 each defining a control rod port 163. The connection between the plates 141 of the frame 14 and the plates 161 of the tang 16 are formed by inwardly tapered proximal portions 145 of the two plates 141, which can be seen clearly in FIGS. 15 and 16.

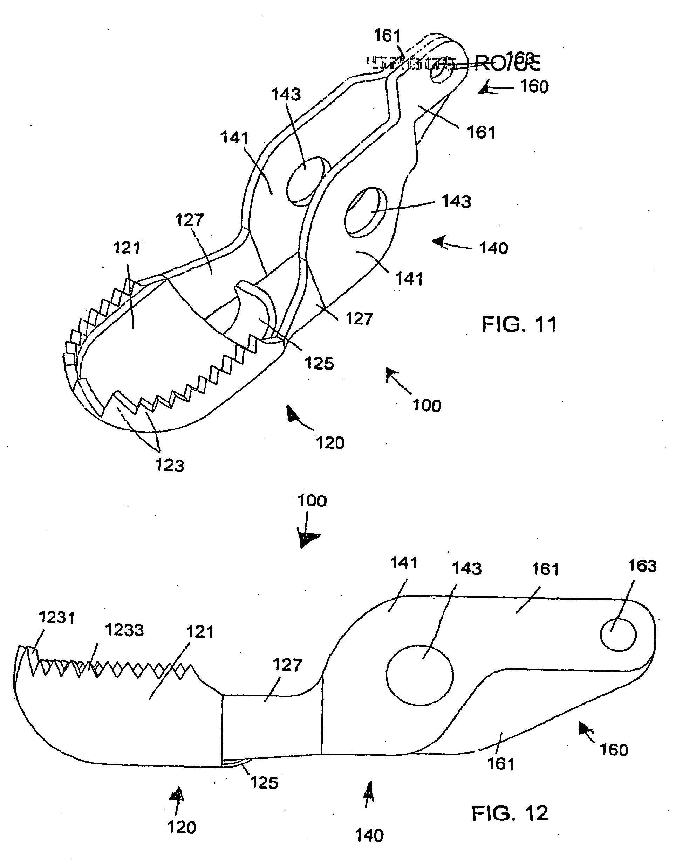

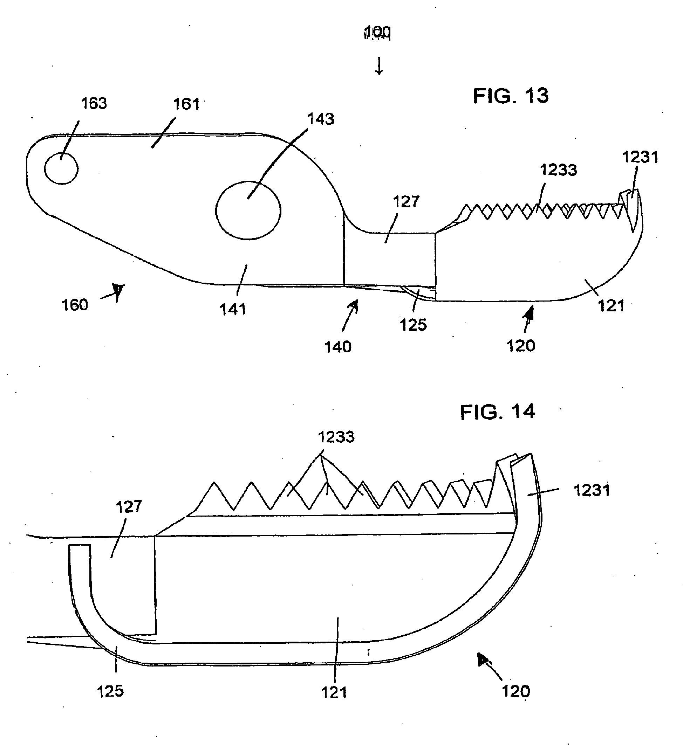

Both the frame 14 and tang 16 have contours to make way for various features of the jaws 10, control wires 3, and/or clevis 20. For example, the proximal arm 127 closer to the viewer of FIG. 10 is shorter than the proximal arm 127 further to the viewer. Also, the plate 161 of the tang 16 closer to the viewer of FIG. 11 is narrower than the opposing plate 161. Compare plates 161 in FIGS. 12 and 13.

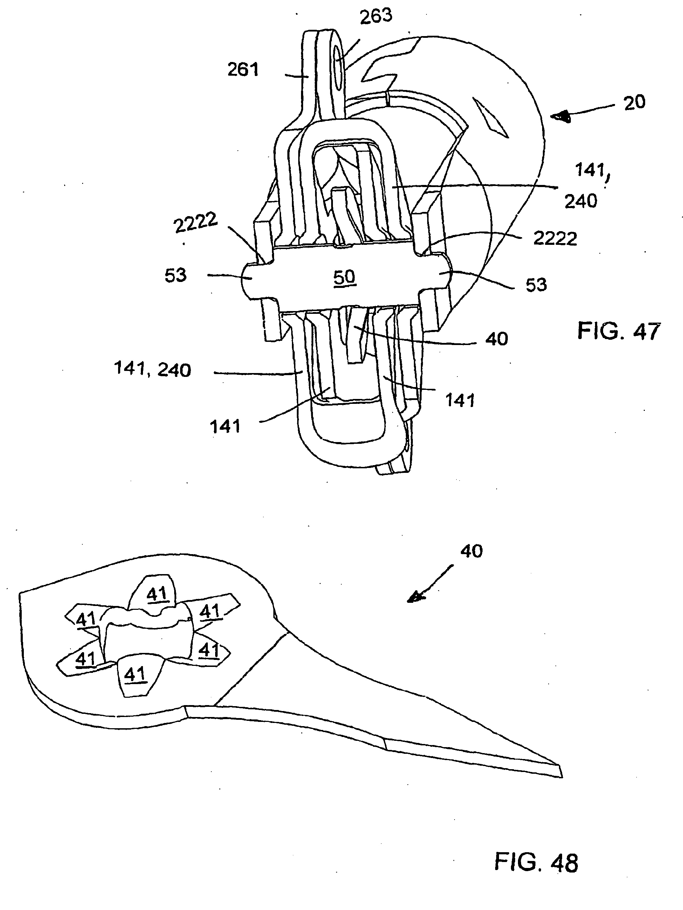

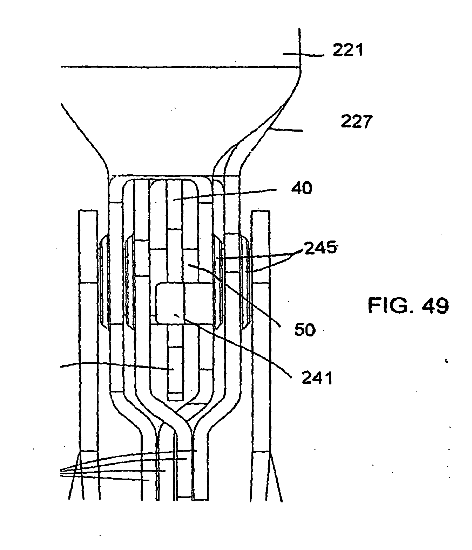

There are significant advantages that are provided by having a dual-part tang 16 as compared to a single tang as shown in FIGS. 1 to 5. Dual tangs are advantageous particularly for a stamped part because the two tangs share loads during use. Accordingly, each tang needs to only be half as strong as compared to a single tang; this also means that thinner materials can be used -- which facilitates the stamping process. The presence of two thin tangs 161 per jaw 100 allows the tangs 161 to be spread wide from one another and interlaced when assembled together. See, e.g., FIGS. 26, 28, 30, and 46 to 50. Because the tangs of a dual tang end effector are not displaced toward the centerline of the part as much as is required by a single tang end effector, there is less plastic deformation of the material during the stamping process, which improves stamping consistency and tool life. Another advantage achieved by spreading the two tangs 161 from one another is that it adds stability to the distal assembly. In the dual tang system, the end effector is supported by two contact points on the axle, spread apart as far as possible from each other. The single tang system supports the end effector by one contact point on the axle. Because of the added support, the dual tang end effector is less prone to cocking (being angularly displaced with respect to the longitudinal axis of the device) than the single tang end effector. This added stability helps maintain end effector, and in the case of a biopsy forceps, tooth, alignment.

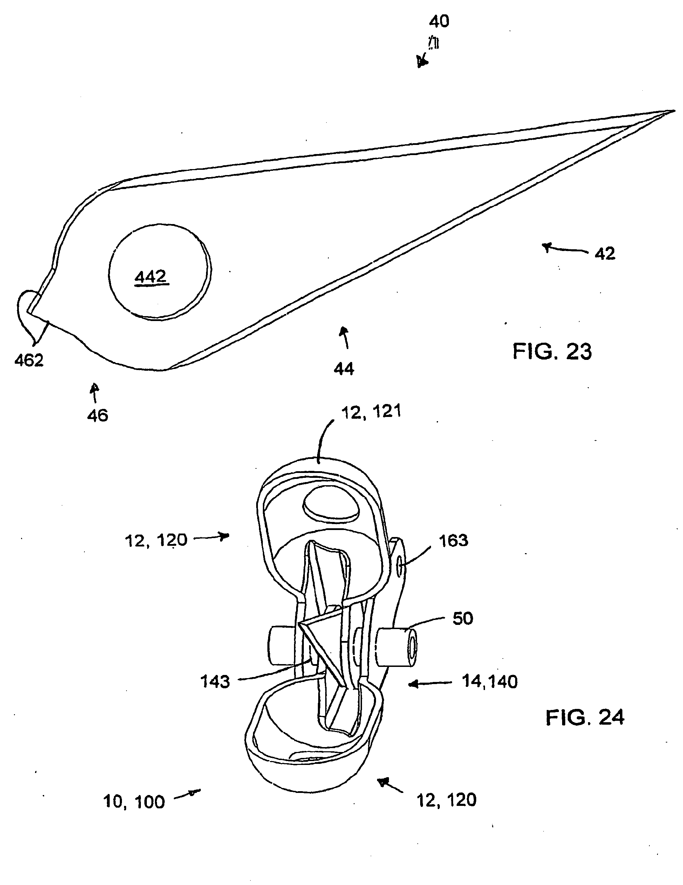

Another important advantage of a dual-part tang 16 lies in the clearance that is formed in between the tangs 161. Positioning the outer plates 141 of the jaws 100 against the clevis and the inner tangs of the jaws 100 against the inner surface of the outer tangs to align the jaws 100 to one another creates a natural gap in the center of the jaw assembly. This gap is best seen in FIGS. 24, 28, and 47 to 50. Therefore, a component, such as the stabilization needle 40, can be added between the jaws 100 within the gap if desired. Single tang configurations need spacers to fill in gaps between the jaws to prevent jaw movement. The dual-part tang 16 of the present invention eliminates the need to fill in the center gap with a component.

The stability afforded by the dual-part tang allows use of thinner and, thus, more flexible materials. This is advantageous for assembly purposes because flexible material tends to give and flex as the device is assembled instead of interfering rigidly with the assembly, as occurs with thicker parts. This benefit allows for more open tolerances during manufacture.

A significant different feature of the one-piece jaw 100 lies in the teeth 123. The distal-most teeth 1231 are larger than the other teeth 1233. The distal-most teeth 1231 are the primary teeth for grabbing and cutting the tissue sample to be obtained. FIG. 16 shows the teeth 1231, 1233 viewed from the distal end of the jaws 100. FIGS. 17 and 18 illustrate the mated nature of the teeth 1231, 1233 and, in particular, show the backward curve of the distal-most teeth 1231. The curve of these teeth 1231 is beneficial because the rearward angle helps them to hook into the tissue better and prevent the teeth from sliding on the tissue while closing the jaws. It also shields the sharp tips of the teeth from the outer surface of the jaws when the jaws are closed, thus making the outer surface smoother and less traumatic to the working channel of the endoscope while advancing the device therethrough.

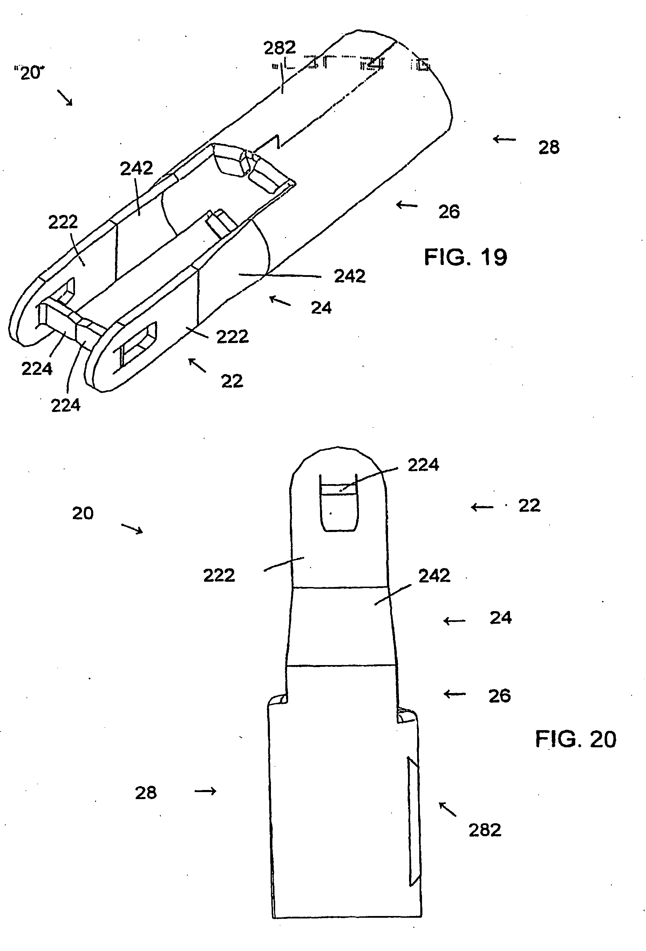

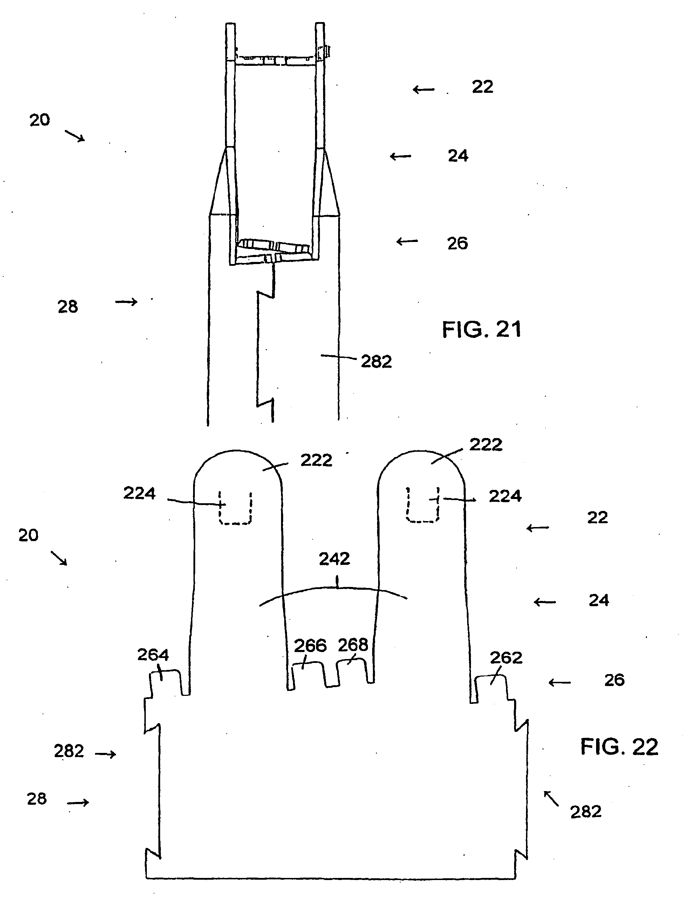

FIGS. 19 to 22 illustrate the preferred embodiment of the clevis 20 of the present invention. To best understand the features of the clevis 20, reference will be made to FIG. 22, in particular, which is the stamped form that, after processing, is shaped into the final clevis 20 illustrated in FIGS. 19 to 21. The clevis 20 has a number of different portions, all of which are integral because the clevis is formed, originally, from the stamped part shown in FIG. 22. The distal-most portion of the clevis 20 is the pivot portion 22. A control portion 24 is adjacent the pivot portion 22 in a proximal direction. A coil-holding portion 26 is adjacent the control portion 24 in a proximal direction. Finally, the coil-retaining portion 28 is adjacent the coil-holding portion 26 at the proximal end of the clevis 20.

Preferably, all of distal components (including jaws 100, clevis 20, and needle 40 (see, e.g., FIG. 23)) are of stainless steel, either work hardened or hardenable by heat treatment to improve the mechanical properties. Use of thin, heat-treatable stainless steel in the surgical end effector has several important advantages. First, the stamping process can be performed when the material is in its pre-heat-treated or in an intermediate, ductile, heat-treat condition. In this formable condition, the material is easily shaped by conventional stamping and forming processes. Once formed to its final shape, the part can be heat treated to improve mechanical properties such as yield strength, ultimate strength, and hardness. By selecting the alloy properly, such as 17-7 precipitation hardening stainless steel, or Carpenter Technology Corporation's Carpenter Custom 455^{™} or Custom 465^{™}, and controlling the heat-treating process (time, temperature, ramp-soak-cool cycles), a combination of high strength and elongation before fracture can be achieved. The preferred materials for the distal components are precipitation hardenable stainless steels such as UNS S17700 (17-7), UNS S17400 (17-4), and age-hardenable stainless steels such as UNS S45500 (Carpenter 455) and UNS S46500 (Carpenter 465). These materials offer the best corrosion resistance of the hardenable stainless steels. The precipitation hardenable alloy 17-7 is of particular interest because, unlike some of the other alloys listed above, 17-7 is austenitic in structure in its annealed state. Thus, there is an improvement in its ability to be formed. Martensitic stainless steels such as 420 or 440 can also be used, but they are not as desirable because their corrosion resistance is not as good as the other alloys mentioned herein.

A second advantage to using such materials is that the distal parts can each be constructed from a thinner material because strength will be added through the heat-treating process. Using thinner material allows for sharper cutting edges in the case of biopsy forceps jaws, for example.

Finally, the ability to use a thinner material affords the stamping die designer more latitude in construction of the die and also improves process capability. There is less strain and subsequent material flow in a thinner section part when bent or formed into the same shape as a thicker section part. This characteristic improves consistency of forming and puts less strain and wear on the die.

With regard to the clevis 20, when the material is of the kind of steel mentioned and when the arms 222, 242 of the control portion 24 are bent outward to load the jaws 10, 100 therein, the arms 222, 242 spring back to their original, parallel orientation such that the tapered ends of each axle half 224 meet without substantial gap therebetween.

The pivot portion 22 has two substantially parallel axle plates 222. After stamping the clevis part shown in FIG. 22, these axle plates 222 are further stamped on the dashed lines shown in FIG. 22 to produce an axle half 224 in each of the axle plates 222. The stamp to produce the halves 224 is a three-sided cut that forms a tab, which after formation by bending inward, rests in a position orthogonal to the plane defined by the respective axle plate 222. Thus, when the stamped part of FIG. 22 is shaped to place the axle plates 222 parallel to one another, the axle halves 224 will lie in a straight line and, therefore, form a bearing upon which the jaws 10, 100 pivot. The ends of the axle halves 224 each have a chamfer so that the axle 50 (see FIG. 24) passing through the pivot port 143 can pass easier and with less outward bending of the control portion 24 when the jaws 10, 100 are installed.

The control portion 24 includes two control plates 242, which have a length sufficient to allow the control parts (the frame 14, 140, the tang 16, 160, and the wires 3) to move without hindrance as they are used to control the opening and closing of the jaws 10, 100. The control plates 242 transition from a substantially flat and planar distal portion to a rounded proximal portion that is adjacent the coil-holding portion 26. When the axle 50 and the jaws 10, 100 are installed on the axle halves 224, the control plates 242 receive most of the outward bending forces. But, because the material of the clevis 20 is sufficiently bendable, the stretching of the two control plates 242 away from one another does not stress the control plates 242 sufficient to plastically deform them or only plastically deforms them to a minimal, non-damaging extent. Thus, the control plates 242 spring back into the position shown in FIGS. 19 to 21 when the jaws 10, 100 are installed.

The coil-holding portion 26 is shaped in an important way. The coil 2 is formed of a rod that is in the form of a spiral where each turn rests on the previous one and the next one. Because of this construction, the last turn does not define a plane. In fact, the longitudinal distal end of the last 360-degrees of the coil is constantly changing. It is important to have the coil be very secure. If the distal stop for receiving the distal end of the coil 2 was planar, then only a portion of the last coil turn would contact the distal stop. To make sure that the last coil turn is contacted properly and securely, the stamped part of the clevis 20 is formed with four coil-holding tabs 262, 264, 266, 268. Each of the tabs is created to be at a different longitudinal distance that follows the pitch of the wire forming the coil 2. Accordingly, when the stamped clevis 20 of FIG. 22 is formed into the part shown in FIGS. 19 to 21, the four tabs 262, 264, 266, 268 are not orthogonal to the longitudinal axis of the clevis 20. Instead, the tabs 262, 264, 266, 268 travel along the spiral path of the coil 2, as shown most clearly in FIGS. 21 and 32 to 34. In order from proximal to distal, the tabs include the first tab 262, the second tab 264, the third tab 266, and the fourth tab 268. These tabs 262, 264, 266, 268 are shown in FIG. 22 as beginning on the far right of the stamped clevis 20. The order illustrated is not required. The first tab 262 can begin on the far left, for example. However, to insure that the tabs 262, 264, 266, 268 follow the shape of the coil, the tabs 262, 264, 266, 268 must be in order (first to fourth) that corresponds to the turn of the coil (left to right if clockwise and right to left if counter-clockwise).

The coil-retaining portion 28 is the tube in which the distal end of the coil 2 is clamped, preferably, by crimping, and secured. If the coil-retaining portion 28 is sized to fit the outer circumference of the coil 2 very closely, then no additional securing device is needed. However, it is important for the clevis 20 to not fall off of the coil 2 and for the clevis 20 to not deform from the final shape shown in FIGS. 19 to 21. Therefore, the stamped clevis 20 of FIG. 22 is formed with a connection device 282 that prevents the circular coil-retaining portion 28 from moving out of its circular shape. It is preferable if the clevis 20 can be formed into the part shown in FIGS. 19 to 21 without connecting the ends together with some external measures. To do so, the connection device 282 uses mechanical measures to connect the right and left ends shown in FIG. 22. The preferred embodiment of the connection device 282 is the dovetail connection shown in FIGS. 19 to 22. With the dovetail, the connection of the right and left ends of the coil-retaining portion 28 are secured to one another merely by mechanical measures.

When the jaws 10, 100 are opened and pressed against tissue to be sampled, it has been found to be beneficial to have a central pin or spike inside the area of the jaws 10, 100 that will house the tissue sample after closing of the jaws 10, 100. The present invention includes a special self-centering blade 40 that is to be located between the jaws 10, 100 inside the cavity formed by the mouth 12, 120 of the jaws 10, 100. As shown in FIG. 23, the blade 40 has a distal cutting area 42, an intermediate pivot area 44 having a pivot bore 442, and a proximal centering device 46 with two centering control surfaces 462. FIGS. 24 and 25 illustrate how the blade 40 is installed between and in the jaws 10, 100.

The preferred cutting area 42 is shown in FIG. 23 and is in the shape of a triangular, double-edged spike. Thus, when tissue is captured in the cavity of the mouth 12, 120, the blade 40 bisects the tissue along the length of the blade 40. FIGS. 24 and 25 show the blade 40 within the jaws 10, 100 and mounted coaxially with the jaws 10, 100 on the axle 50. It is noted that the axle 50 does not extend past the right side of the frame 14, 140 when inserted between the axle plates 222 of the clevis 20 as shown in FIGS. 24 and 25. The axle 50 is shown longer in FIGS. 24 and 25 for clarity purposes only. In the preferred embodiment, the axle 50 is approximately flush with the outer planar surfaces of the frame 14, 140 or extending just a little bit out from that plane so that the frame 14, 140 does not rub against the inner surfaces of the axle plates 222 of the clevis 20 when pivoting thereon.

As set forth above with regard to the tangs 160 and the wires 3, the tangs 16, 160 are shaped to not interfere with the pivoting of the one-piece jaw 100. FIG. 26 illustrates an embodiment of the connection between the control wire 3 and the tang 150 of each jaw 100. The embodiment shown in FIG. 26 does not include the centering blade 40, however, with appropriate shaping of the transition between the frames 140 and the tangs 160, a space can be left in the center of the two jaws 100 so that the blade 40 can fit on the axle 50 and in the center of the jaws 100 as indicated, for example, along the dashed line 52 on the axle 50. The wires 3 extend longitudinally up to the control rod port 163, bend ninety degrees to enter into the control rod port 163, extend through both the control rod ports 163 of each of the two parallel plates 161 along an orthogonal portion 32, exit the control rod port 163 of the second parallel plate 161, and bend ninety degrees to, thereby, engage the tang 160 without the possibility of falling out of the ports 163. It is noted that the fit between the ports 163 and wires 3 is sufficiently loose to permit the tangs 160 to rotate freely about the orthogonal portion 32.

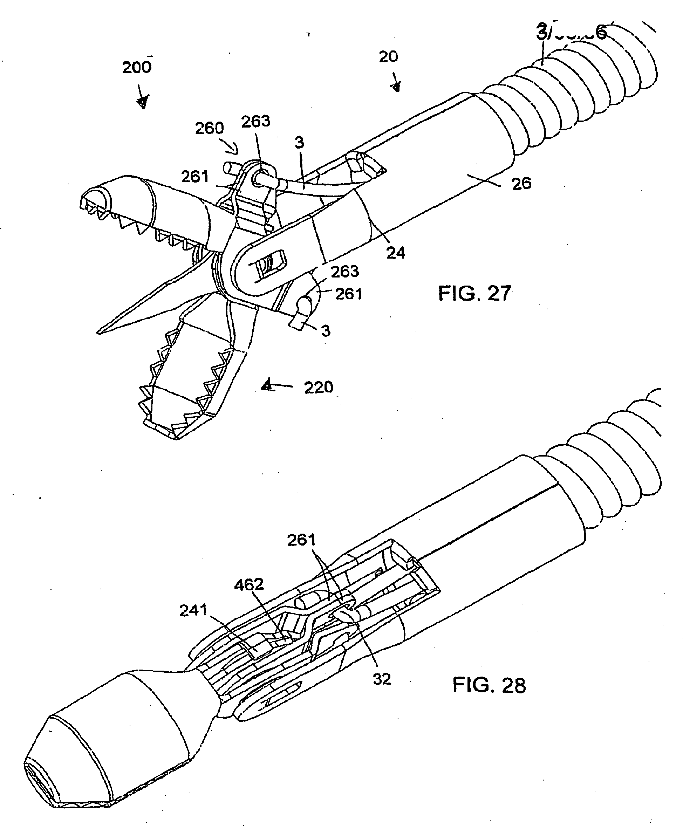

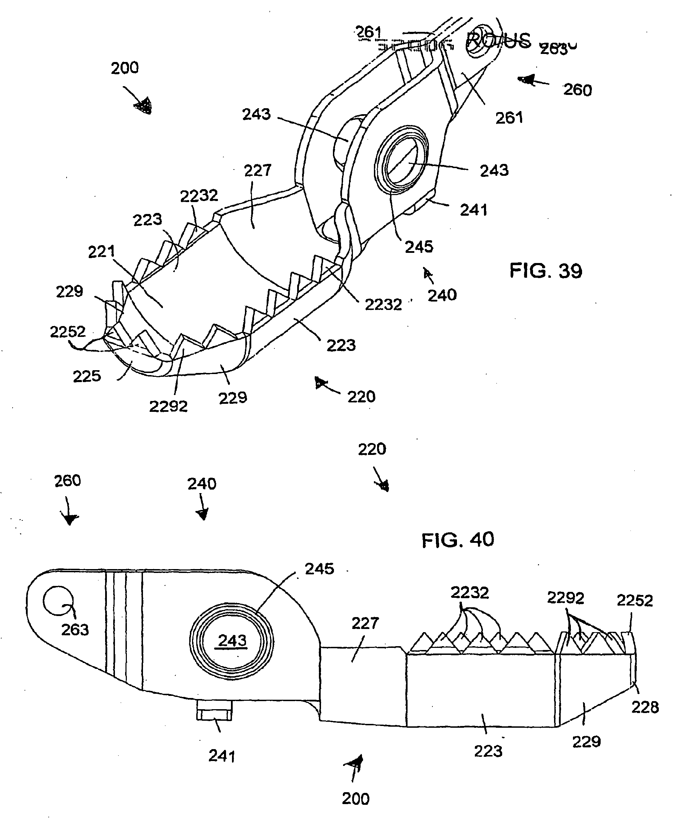

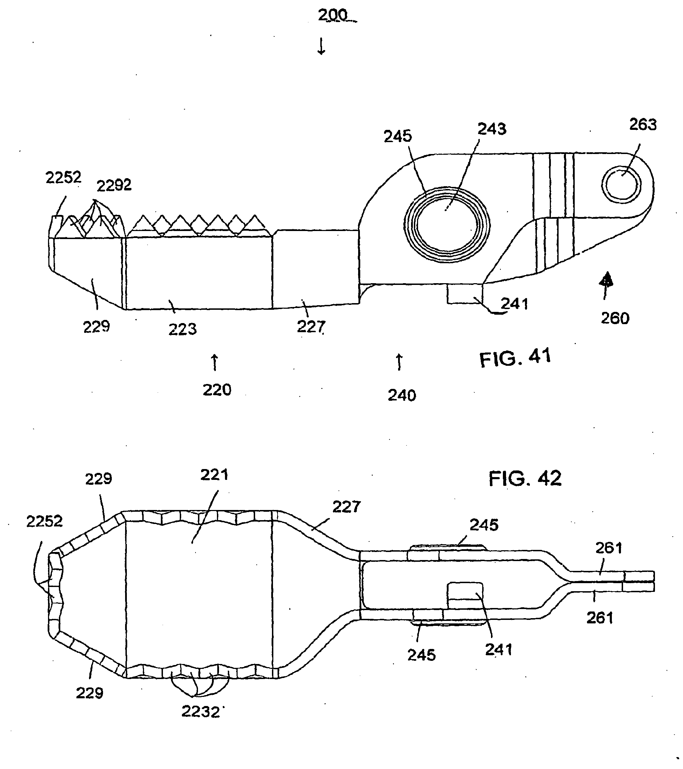

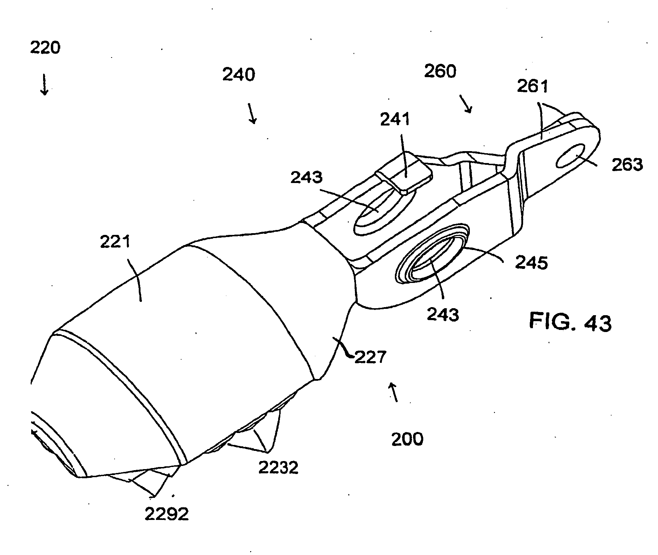

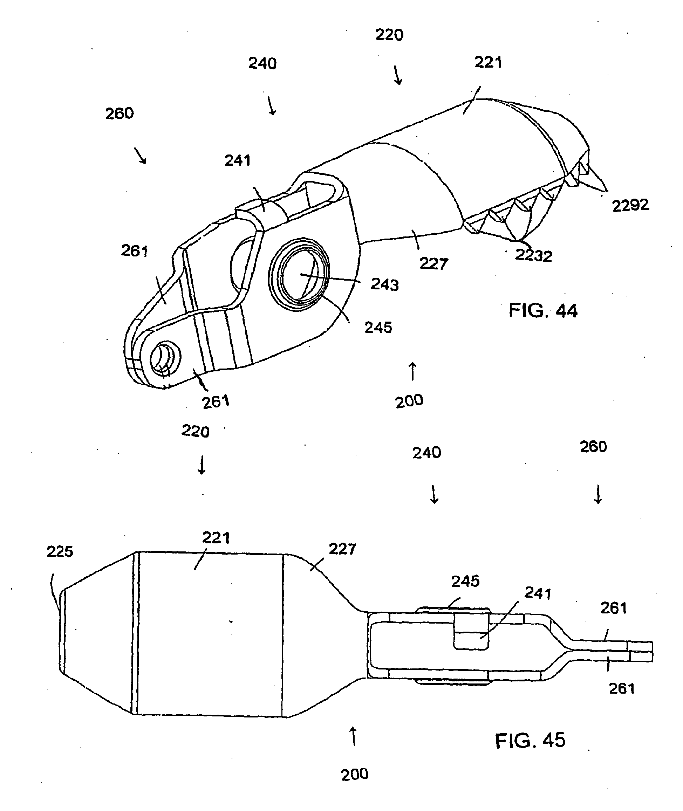

FIGS. 27 to 29 illustrate the preferred embodiment of the end effector 200 of the present invention. The clevis 20 and coil 2 are the same as in the other embodiment but the jaws 220 are different and will be explained in further detail with regard to FIGS. 39 to 50. The clevis 20 of FIGS. 27 et seq., however, illustrates the coil-retaining portion 28 without the dovetail connection device 282.

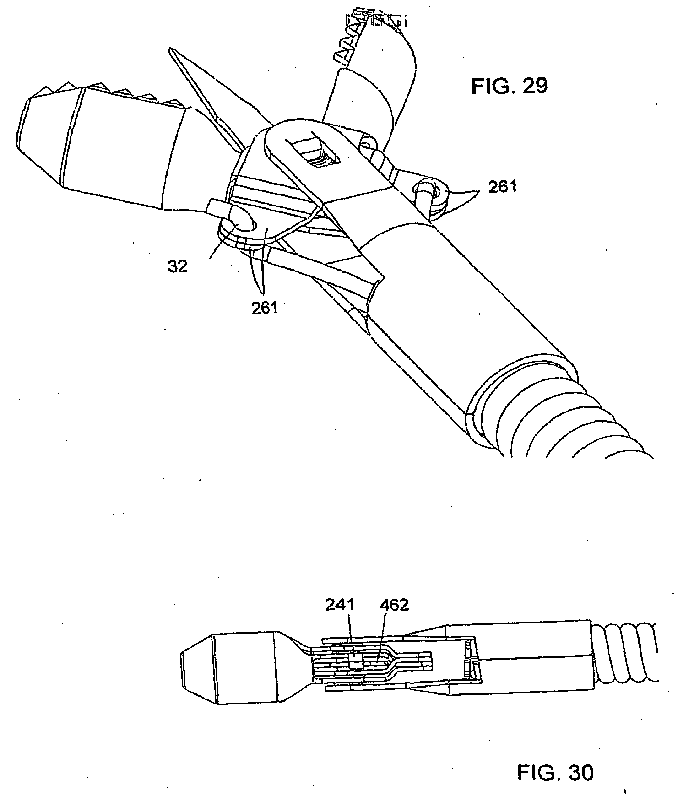

As can be seen, the control wires 3 emerge from the distal end of the coil 2 and from the coil-holding portion 26 of the clevis 20 at the control portion 24 of the clevis 20. The wires 3 bend ninety degrees to enter into the control rod port 263, extend through both the control rod ports 263 of each of the two parallel plates 261 along an orthogonal portion 32 (see FIG. 28), exit the control rod port 263 of the second parallel plate 261, and bend ninety degrees to, thereby, engage the tang 260 without the possibility of falling out of the ports 263. As seen, in particular, in FIGS. 27 and 29, the fit between the ports 263 and wires 3 is sufficiently loose to permit the tangs 260 to rotate freely about the orthogonal portion 32.

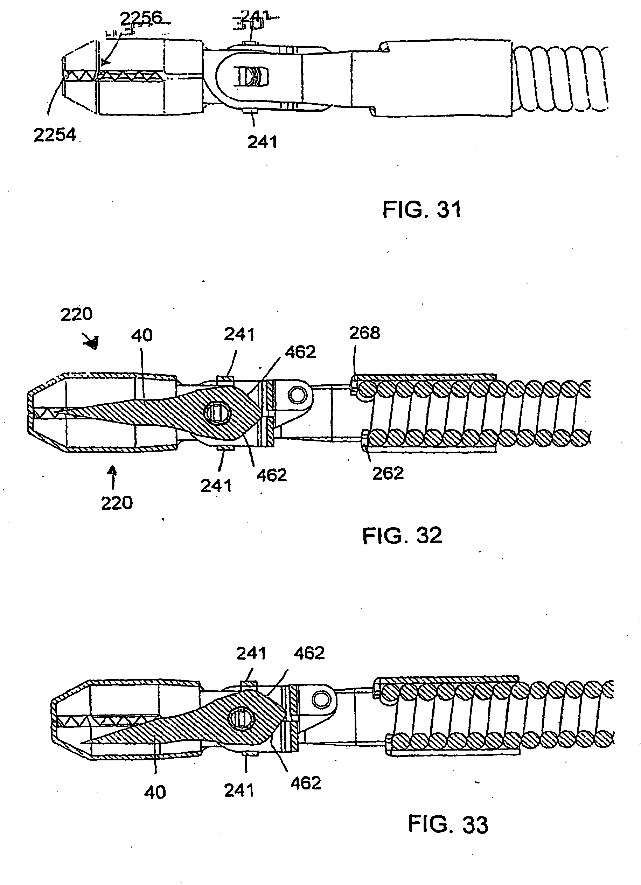

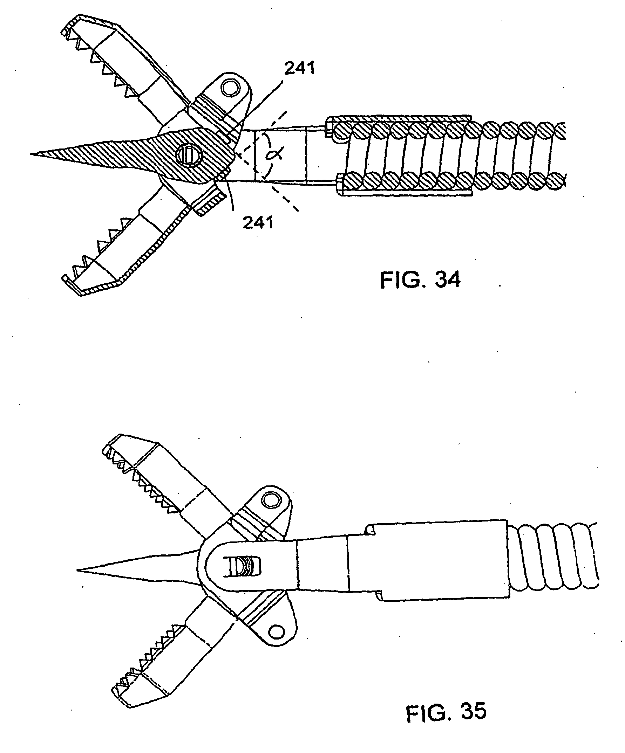

One of the two centering control surfaces 462 of the proximal centering device 46 of the blade 40 is shown clearly in FIGS. 28 and 30 to 34. The control surface 462 interacts with a blade control tab 241 on the frame 240. The cross-sectional view of FIGS. 32 to 34 shows the interaction between control surface 462 and the blade control tab 241. When the jaws 220 are closed, the control surfaces 462 do not interact with the blade control tabs 241. The movement of the blade 40 shown in FIGS. 32 and 33 illustrates the free pivot of the blade 40 when the jaws 220 are closed. In contrast, when the jaws 220 are opened past a given extent, the control surfaces 462 interact with the blade control tabs 241 and, as shown in FIG. 34, rest directly thereupon so that the control surface 462 lies on and parallel to the respective contacting surface of the blade control tab 241. Because of this configuration, the blade control tab 241 also acts as a jaw limiter, which defines the greatest extent that the jaws 220 can be opened. Simply put, the angle α defined by the two control surfaces 462 (see FIG. 34) defines the maximum opening angle of the jaws 220. If the angle α increases from that illustrated in FIG. 34, then the jaws 220 can open more and if the angle α decreases, then the jaws 220 can open less. FIG. 35 illustrates the elevational view of the jaws 220 of FIG. 34.

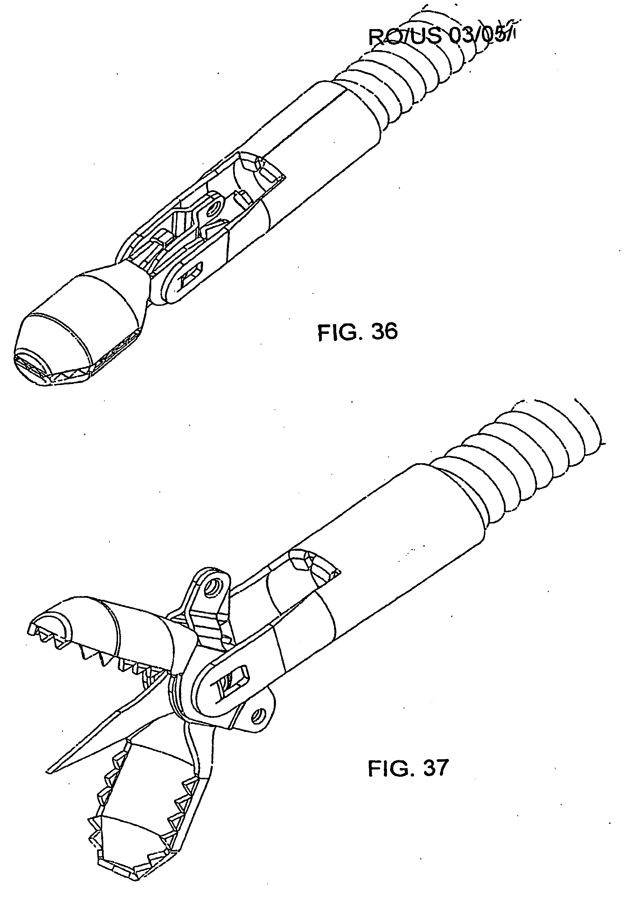

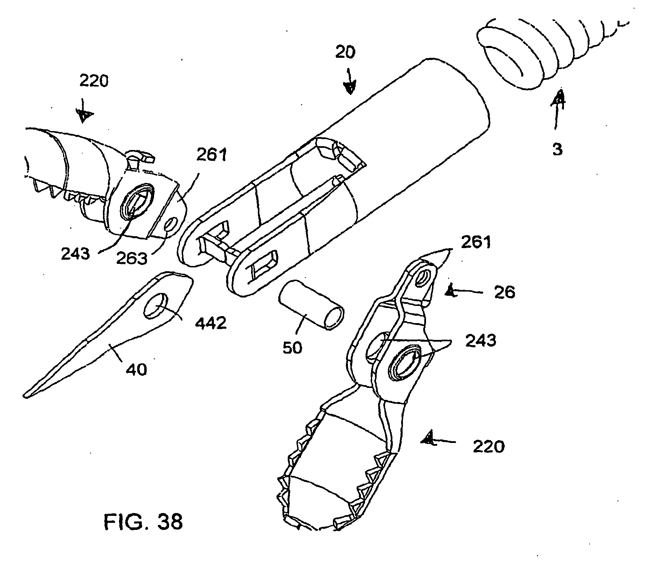

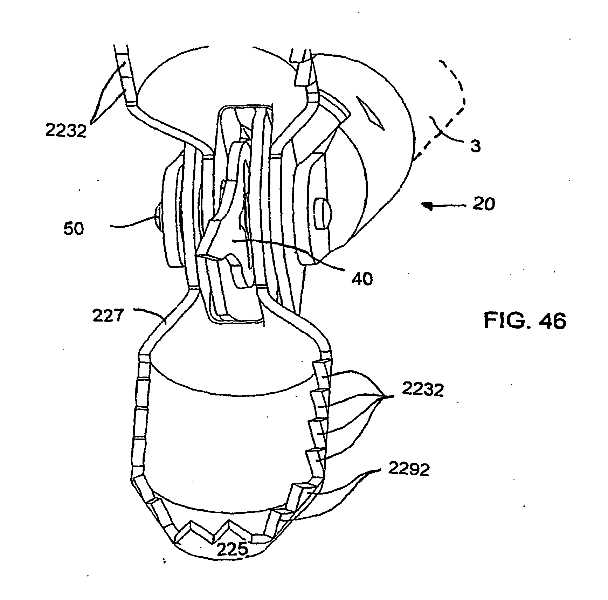

FIGS. 36 and 37 show views of the preferred embodiment similar to FIGS. 27 and 28 but without the control wires 3, and FIG. 38 illustrates, in an exploded view, the various parts of the end effector of FIGS. 27 to 50. To assembly the end effector of the present invention, one set of the parallel plates 261 of one tang 26 is pulled apart and the two jaws 220 are nested. See, e.g., FIGS. 46 to 50. The blade 40 is placed in the center of the two jaws 220 and the axle is passed through the pivot ports 243 of each and the pivot bore 44 of the blade 40. Then, the two axle halves 224 are pulled apart so that the axle 50 can fit over one of the axle halves 224. One opening of the axle 50 is placed all the way on one axle half 224 and the other axle half 224 is allows to spring back to, with the aid of the chamfer on the distal end of the other axle half 224, enter into the second, opposite opening of the axle 50. The control wires 3 are, then, inserted through the control rod ports 263 to, thereby, fix the jaws 220 in position in the clevis 20.

FIGS. 27 to 50 illustrate the preferred embodiment of the jaws 220 of the present invention. As can be seen in all of FIGS. 27 to 50 and, especially, in FIGS. 39 to 50, the mouth portion 221 is angular in its configuration. The two lateral sides 223 are substantially parallel to one another and are substantially linear and the front face 225 is substantially orthogonal to the lateral sides 223 and is also substantially linear. Respective angled sides 229 connect the lateral sides 223 to the front face 225. Like the lateral sides 223 and the front face 225, the angled sides 229 are substantially linear. The proximal arms 227 connect the lateral sides 223 to the frame 240.

In FIGS. 27 to 50, the lateral sides 223 are shown with teeth 2232, the front face 225 is shown with teeth 2252, and the angled sides 229 are shown with teeth 2292. However, any of these teeth 2232, 2252, 2292 can be removed. In one preferred embodiment, the teeth 2232, 2292 of the respective sides 223, 229 can be configured to form a space therebetween to create gaps at the junctions of the lateral-angled sides and the angled side-front face. Such a configuration allows the captured tissue in the mouth portion 221 to flow or bulge out of the resulting spaces and, therefore, makes room for more tissue to be captured as the biopsy sample. Gaps at these junctions also provides the second benefit of minimizing edges that can scrape or otherwise damage the working channel of an endoscope as the end effector passes therethrough. These gaps will be discussed in further detail below.

Each outer surface of each frame 240 is formed with a protruding ring 245 surrounding the pivot port 243 for receiving the axle 50. This ring 245 permits the nested parts -- needle 40, jaw 220, jaw 220 -- to pivot freely with respect to one another about the axle 50.

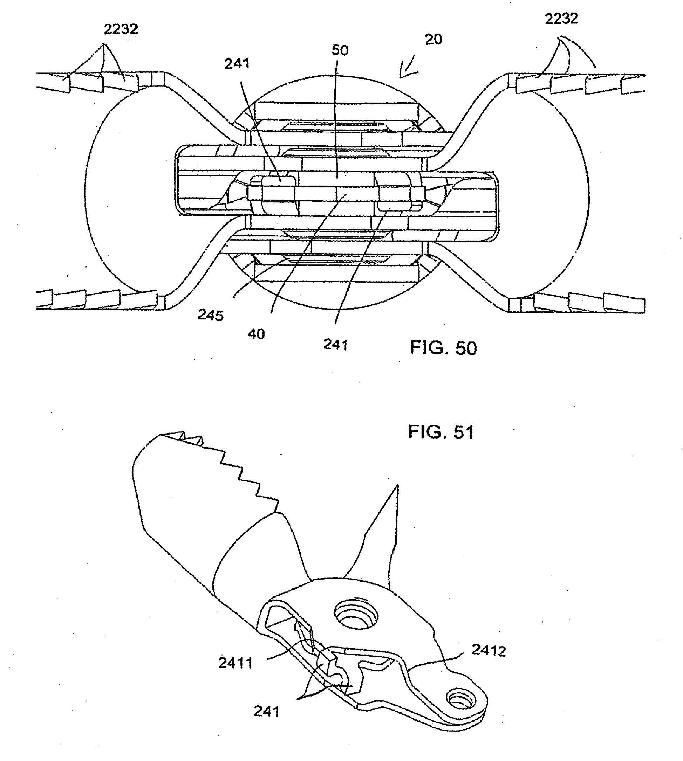

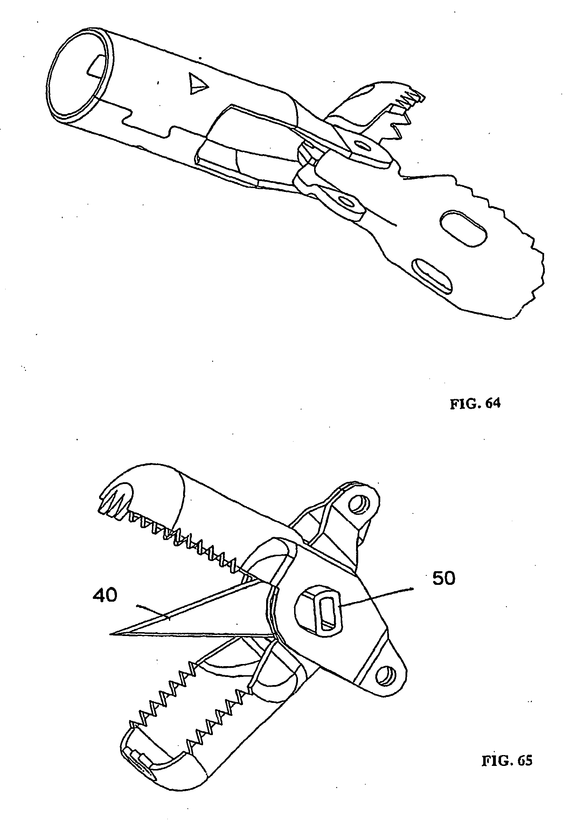

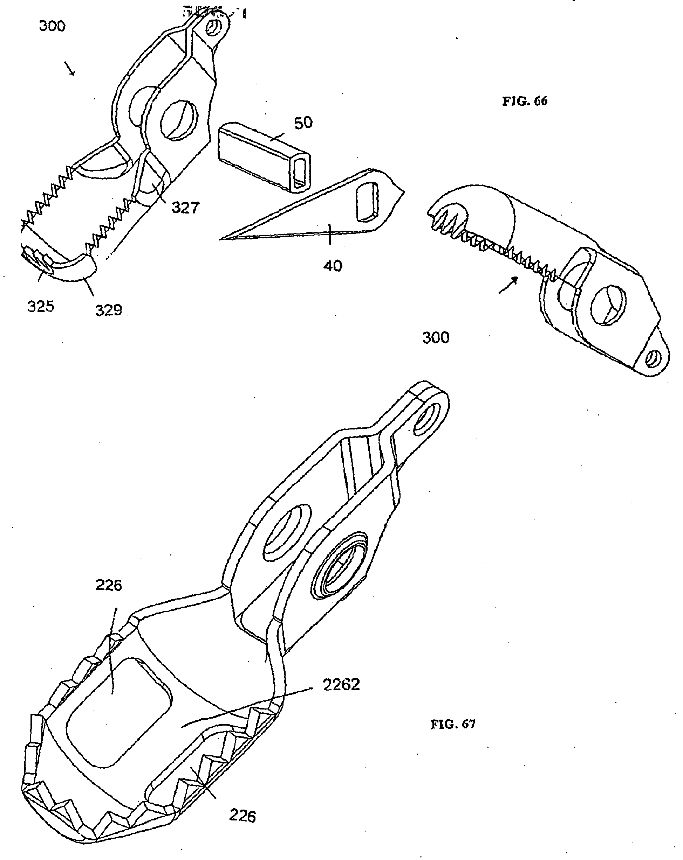

The jaw configuration of FIGS. 27 to 50 is not the only variation for the frusto-conical distal end 229, 225 of the jaws 220. As shown in FIG. 51, for example, the distal end can be bi-frusto-conical and have two angled sides portions, a proximal portion 229' and a distal portion 229''. Also, as shown in FIGS. 65 and 66, the frusto-conical portion 229 can have a curved or frusto-hemispherical shape.

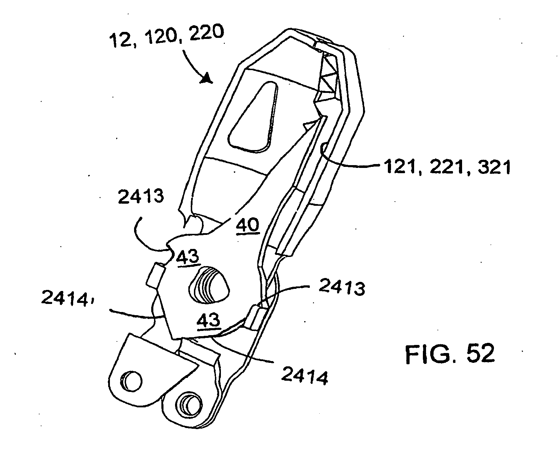

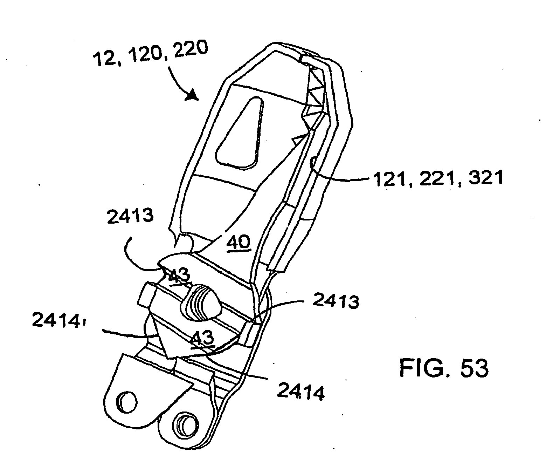

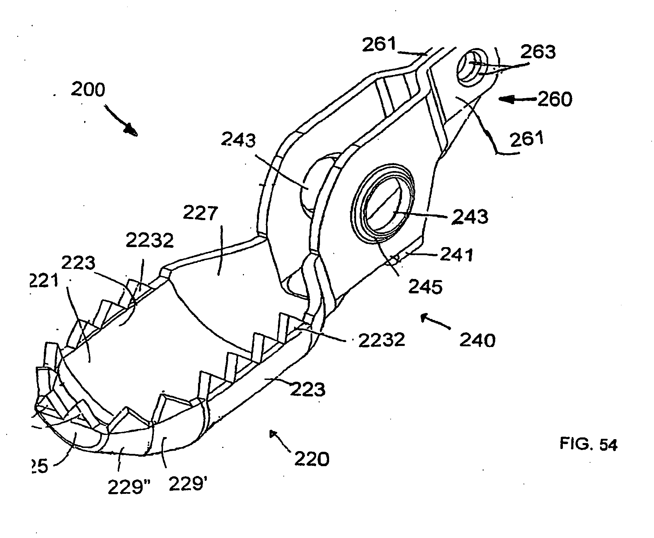

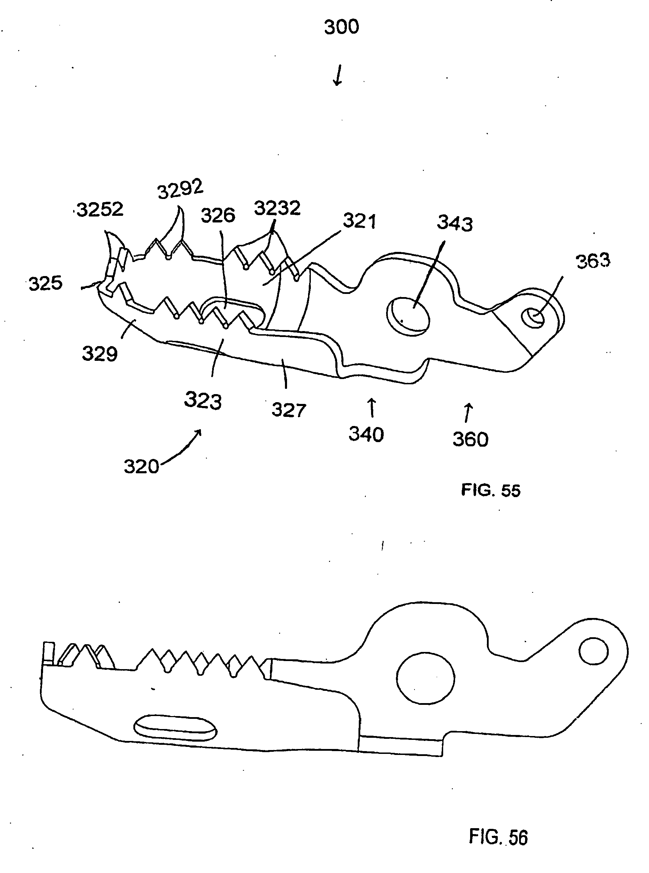

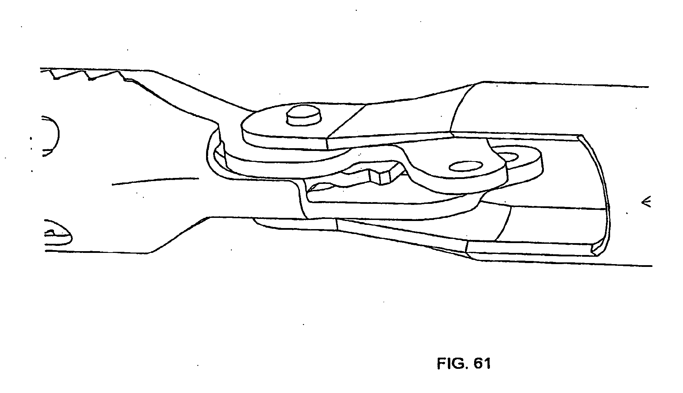

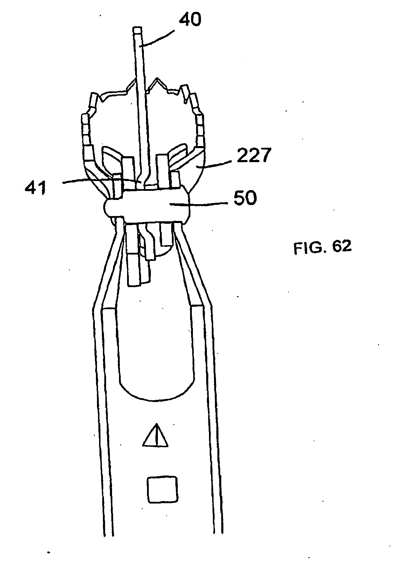

As set forth above, the two tang embodiment is a preferred configuration. However, like FIGS. 1 to 5, the embodiment of FIGS. 27 to 50 can also be constructed,with a single tang. Such a variation is illustrated in FIGS. 52 to 64.

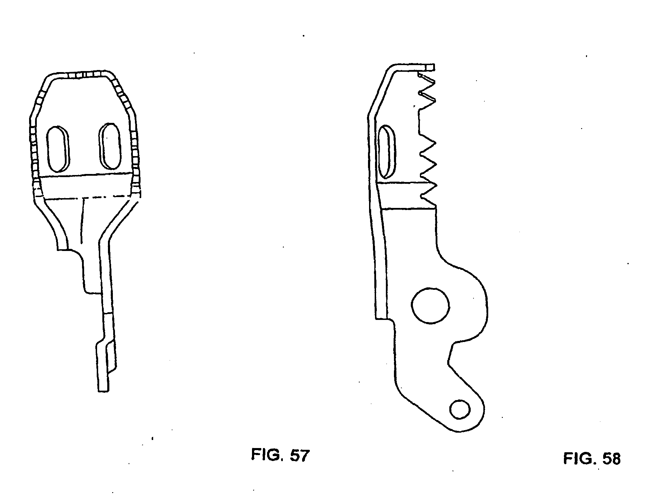

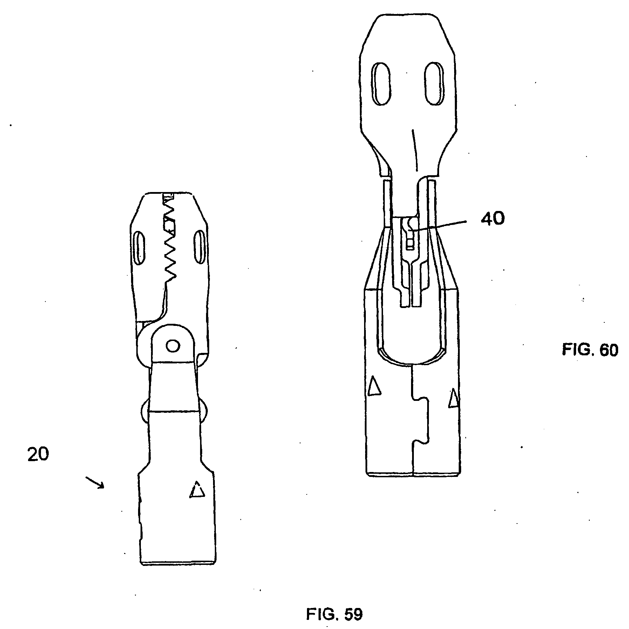

FIGS. 52 to 57 illustrate the alternative embodiment of the jaw 320 of the end effector 300 having a single tang 360. Teeth 3232, 3252, 3292 can be present or absent from any of the edges. In the illustrated embodiment, the two lateral sides 323 are shown with four teeth 3232 each and the frusto-conical portion 329 has one or two teeth 3292 on the two edges, respectively. Finally, the front face 325 is shown with two teeth 3252. The opposing jaw 320, shown particularly in FIG. 58 (and also in FIGS. 60 to 64) can be configured without teeth or with teeth that interdigitate with the teeth of the jaw 320 shown in FIG. 52.

As can be seen in all of FIGS. 52 to 64 and, especially, in FIGS. 52 and 58, the mouth portion 321 is angular in its configuration. The two lateral sides 323 are substantially parallel to one another and are substantially linear and the front face 325 is substantially orthogonal to the lateral sides 323 and is also substantially linear. Respective angled sides 329 connect the lateral sides 323 to the front face 325. Like the lateral sides 323 and the front face 325, the angled sides 329 are substantially linear (but can be curved as shown in FIGS. 65 and 66). The proximal arms 327 connect the lateral sides 323 to the frame 340.

In FIGS. 52 to 64, the lateral sides 323 are shown with teeth 3232, the front face 325 is shown with teeth 3252, and the angled sides 329 are shown with teeth 3292. However, any of these teeth 3232, 3252, 3292 can be removed. In one preferred embodiment, the teeth 3232, 3292 of the respective sides 323, 329 can be configured to form a space therebetween to create gaps at the junctions of the lateral-angled sides 323 and the angled side-front face 325. Such a configuration allows the captured tissue in the mouth portion 321 to flow or bulge out of the resulting spaces and, therefore, makes room for more tissue to be captured as the biopsy sample. Gaps at these junctions also provides the second benefit of minimizing edges that can scrape or otherwise damage the working channel of an endoscope as the end effector passes therethrough. These gaps will be discussed in further detail below.

The features of the tang 360 and the tang interface to the blade 40 are similar to the dual-tang configuration of the stamped jaw shown in FIGS. 10 to 18 and 27 to 51 and, therefore, will not be repeated herein.

It is noted that each of the distal end portions of the present invention is illustrated as being frusto-conical. This, however, is not the only configuration for the distal-most end of the end effector. In an alternative embodiment, the distal-most end (i.e., 225, 325) can come to a point, can be curved, or can be entirely non-existent. As such, in one alternative embodiment of the front face 225, 2252, 325, 3252, either the teeth 2252, 3252 or both the teeth 2252, 3252 and the front face 225, 325 can be removed to provide additional spaces in which captured tissue in the mouth portion 221, 321 can flow or bulge out.

Like the jaw 220, each outer surface of each frame 340 can be formed with a protruding ring surrounding the pivot port 343 for receiving the axle 50, which permits the nested partsneedle 40, jaw 320, jaw 320 -- to pivot freely with respect to one another about the axle 50.

It is advantageous to use annealed, thin sheet metal when forming the end effectors of the present invention by stamping. Such thin sheet metal allows tighter bends and finer detail to be produced during the stamping process. Annealed material is easier to form and cut and produces less tool wear. Once formed from the thin stock, the end effector jaw may not have the required mechanical properties without secondary processing. Secondary processing can greatly enhance the mechanical properties of such sheet metal and examples of such processing include: heat treatment, age hardening, case hardening, ion implantation, carbon-nitriding, cold working or combinations of these. In the case of biopsy forceps jaws formed from 17-7 precipitation hardenable stainless steel, post processing (precipitation hardening) to increase the mechanical properties of the material is required as the ratio of the diameter of the end effector to the thickness of the material approaches 11:1. Preferably, the ratio for a flexible, endoscopic instrument (such as a biopsy forceps) is between approximately 30:1 and approximately 11:1, in particular, between approximately 20:1 and 11:1, and specifically, at approximately 17:1 or at approximately 11.4:1.

FIGS. 65 and 66 show a further embodiment of the jaw assembly not according to the present invention. The jaws 300 have a curved intermediate section 329 flanked by linear sides 323 and a linear front face 325. In this embodiment, the axle 50 has an outer rectangular shape with rounded ends. The outer shape corresponds to the shape of the axle port in the spike 40 so that the spike remains in a position corresponding to the orientation of the axle 50.

FIGS. 67 to 70 show yet another embodiment of the jaw assembly of the present invention. This embodiment expands upon the ability of the jaws 220 to ensure that the maximum volume of tissue is captured in the mouth portion 221 of the jaws 220. Many of the features are similar to FIGS. 39 to 45, for example, and, therefore, explanation of similar features is not repeated. In contrast to the embodiment illustrated in FIGS. 27 to 50, the mouth portion 221 and two lateral sides 223 define fenestrations 226. Such a configuration allows the captured tissue in the mouth portion 221 to flow or bulge out of the resulting spaces and, therefore, makes room for more tissue to be captured as the biopsy sample.

The fenestrations 226 need not merely be cut at right angles to the plane of the stamped jaw 220. The edges of each fenestration 226 can be formed towards the inside in a stamped forceps cup to effectively round the edges thereof and, therefore, decrease the possibility of scope channel wear caused by movement of the forceps inside the endoscope, for example. Two teardrop-shaped or egg-shaped fenestration holes 226 are better than one hole 226 for scope wear because the web 2262 between the holes 226 will be presented to the scope channel as the forceps pass therethrough instead of the edge of the hole being presented to the scope channel.

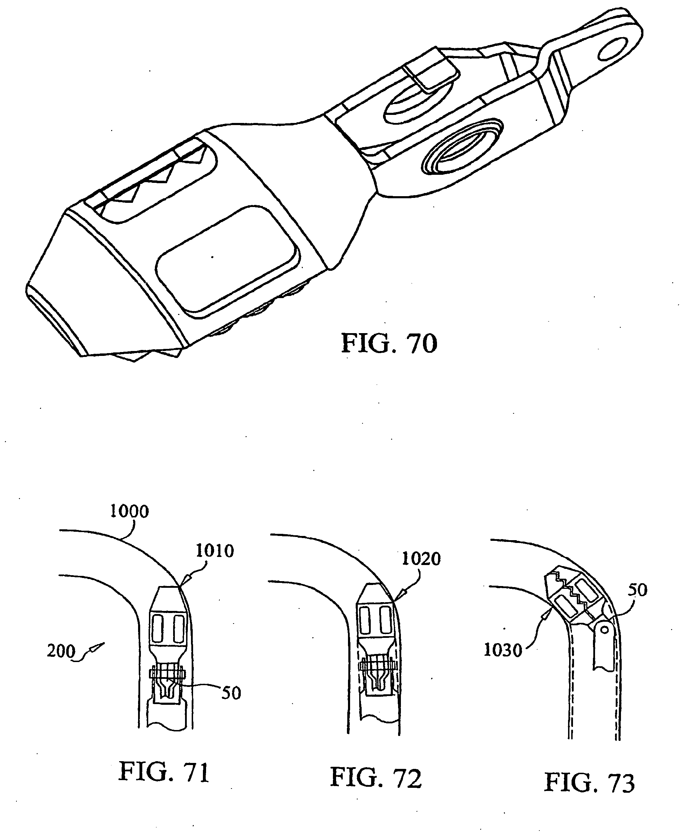

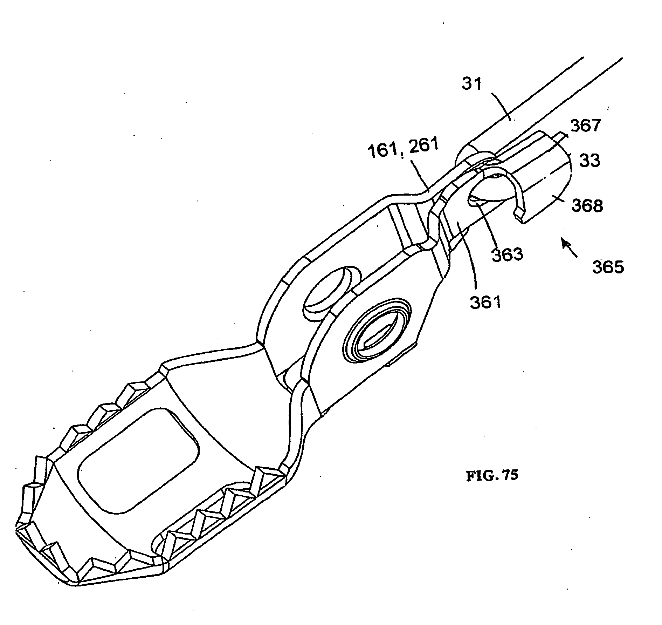

Biopsy forceps cups are typically fenestrated. However, the prior art designs do not reduce chances for endoscope wear, rather, they increase the probability. As set forth above, the exposed edges of the fenestrations 226 (see FIGS. 67 to 72 and 74 to 75) are shaped to minimize such contact. By separating the fenestrations 226 with a center web 2262, only the smooth surface of the web 2262 is in contact with the scope channel as the end effector passes through the channel. See, i.e., FIG. 73. Accordingly, as the end effector 200 impinges upon a curve in the working channel, the intersection of the angled nose edge and the lateral straight edge of the jaw can contact the scope channel. If this intersection includes a tooth edge, the edge can be forced against the wall of the scope channel potentially damaging it. Therefore, it is advantageous to' interrupt the teeth in this transition area to minimize the potential of scope channel wear.

When passing through such a curve, end effectors are forced against the surface of the working channel 1000 as they track around the curve. Similarly, as an end effector of a typical biopsy forceps passes through a working channel, it, too, slides along the interior surface of the working channel. This increased force of sliding can cause endoscope channel wear if the surface of the end effector is rough, or if it has exposed edges -- such as the exposed edges of sides of forceps teeth. Thus, the present invention provides various aspects that decrease the possibility of damaging the working channel of a flexible endoscope.

FIGS. 71 to 73 illustrate the end effector 200 of the present invention passing through a curve in a working channel 1000 of a flexible endoscope. FIG. 71 illustrates a "worst-case" scenario with respect to contact between the'end effector 200 and the channel 1000. Specifically, the bending axis of the end effector 200 - corresponding to the axle 50 (see, i.e., FIG. 24) - is parallel to and lies in the plane defined by the curve of the working channel 1000 (which is also, in this example, the plane in which all of FIGS. 71 to 73 lie). In such a configuration, the end effector 200 substantially cannot bend when it first hits the curve -- shown by first contact point 1010 in FIG. 71 - because the jaws 220 only pivot about the axle 50. Therefore, to traverse the curve, the axle 50 needs to be at least at an angle to the plane of the curve or, ideally, perpendicular to the plane of the curve.

The first contact point 1010 is located at a first tooth gap 2254 on either side of the teeth 2252 on the front face 225. See, i.e., FIG. 31. If a tooth 2252 were located at the gap 2254, then the potentially sharp outer surfaces of the tooth 2252 could damage the inner surface of the working channel 1000. Thus, the first gap 2254 is provided by the present invention. Because the end effector 200 is being pushed further into the curve, as the end effector 200 is traversing the curve, the outside of the coil 2 can bend slightly to place a second contact point 1020 against the interior surface of the working channel 1000. FIG. 72 indicates the second contact point 1020 touching the working channel 1000 and the first contact point 1010 not touching the working channel 1000. Of course, depending on the curve of the working channel 1000, both contact points 1010, 1020 can touch the interior surface of the working channel 1000.

This second contact point 1020 is located at a second tooth gap 2256 between the teeth 2292 and the side teeth 2232 on the angled side 229 and the lateral side 223, respectively. See, i.e., FIG. 31. If a tooth 2232 or 2292 were located at the gap 2256, then the potentially sharp outer surfaces of the tooth could damage the inner surface of the working channel 1000. Thus, the second gap 2256 is provided.

The forceps of the present invention has a relatively long coil 2 that can be rotated somewhat about its longitudinal axis without irreparable deformation. As such, when the end effector 200 traverses through the curve, the rotational resistance is much less than the lateral bending forces imposed in the plane of the axis 50 to, thereby, naturally rotate the end effector 200 inside the working channel 1000 to an orientation shown, for example, in FIG. 73. In such an orientation, the end effector 200 can bend about the axle 50 and, ideally in this example, rotate ninety degrees to place the web 2262 against the inner surface of the curve at third contact point 1030. Because the web 2262 is smooth and curved, it passes through the curve of the working channel 1000 with minimal wear.

Next, the force of passage can be mitigated by decreasing the rigid (non-bendable) length of the end effector so that it tracks around and conforms better to the curves.

Prior art devices include a centering spike that is significantly longer than the spike 40 of the present invention. These prior art centering spikes can even extend through the end effector in a proximal direction well past the clevis. The centering spike of the Radial Jaw^{™} biopsy forceps manufactured by Boston Scientific is one example where the rigid length is approximately two times longer than the spike 40 of the present invention. The presence of such a long centering spike prohibits the prior art end effector from pivoting when the jaws are closed, thus effectively increasing the rigid length of the prior art end effector.

In contrast, the present invention allows the centering spike 40 to freely pivot within and with the jaws 220 to, thereby, effectively decrease the rigid length of the end effector 200 and make passage through the working channel much easier than the prior art end effectors.

FIGS. 27 to 29, 68, and 69 show a first embodiment of the actuating wires or rods 3. In that embodiment, the wires 3 extend longitudinally up to the control rod port 163, 263, bend ninety degrees to enter into the control rod port 163, 263, extend through both the control rod ports 163, 263 of each of the two parallel plates 161, 261 along an orthogonal portion 32, exit the control rod port 163, 263 of the second parallel plate 161, 261, and bend ninety degrees to, thereby, engage the tang 160, 260 without the possibility of falling out of the ports 163, 263. To actuate the forceps, a force is exerted on the rod 3. The S-bend of the wire 3 significantly reduces any possibility that the wire 3 becomes dislodged from the tang 160, 260.

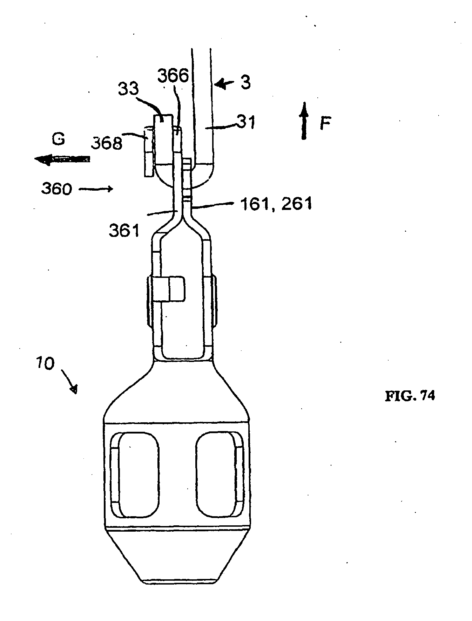

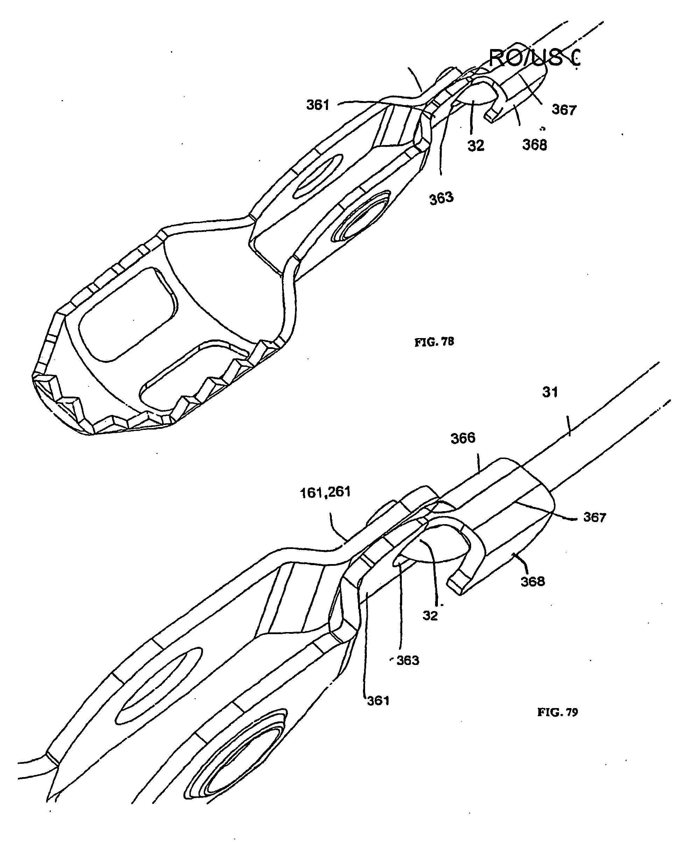

FIGS. 74 to 79 illustrate another embodiment 360 of the tangs 160, 260 and second and third embodiments of the wires 3 shown in the previous figures.

As set forth above, the wire 3 is attached to the jaw 10 to allow the wire 3 to freely rotate and to allow each jaw 10 of the end effector 1 to pivot. The wire 3 must be secured so that it can both push and pull on the tang 160, 260, 360 of the jaw 10 but not become dislodged therefrom. The primary transmission of force occurs during closing of the end effector 1, which is accomplished by pulling on the wire 3 in a distal direction. As tension is applied to the wire 3, the bends in the distal end of the wire 3 that connect the wire 3 to the tang 160, 260, 360 are stressed and can begin to straightenallowing the wire 3 to pull though the control rod port 163, 263, 363 and slip free from the tang 160, 260, 360. This possibility only exists, however, if no measures are taken to prevent such straightening.

In particular, FIGS. 74 to 76 illustrate the second embodiment of the wires 3. In these figures, only one jaw 10 is shown. However, each jaw 10 has its respective wire 3 and, therefore, the description herein need not be repeated. To attach to the jaw 10, the wire 3 extends longitudinally up to the control rod port 363 (from the proximal end of the forceps device) along a first portion 31, bends ninety degrees to enter into the control rod port 363, extends through both the control rod ports 363 of each of the two parallel plates 361 along an orthogonal portion 32, exits the control rod port 363 of the second parallel plate 361, and bends another ninety degrees to extend back in the proximal direction along an third portion 33 and, thereby, engage the tang 360. This second embodiment of the wire 3 significantly reduces any possibility that the wire 3 becomes dislodged from the tang 360.

To effect the reduction of unintended wire 3 removal, one plate 361 of the two parallel plates 161, 261 has a hooded wire support 365. The hooded wire support 365 is configured to provide support to the wire 3 and, thereby prevent removal of the wire 3 from the tang 360. The hooded wire support 365 is substantially U-shaped in cross-section and, therefore, has a vertical first leg 366, a horizontal portion 367, and a vertical second leg 368 opposite the first leg 366. The vertical first leg 366 is integral with the plate 361 and extends therefrom at one edge of a proximal portion of the plate 361(of course it can extend from the other edge or from both edges). The support 365 surrounds the third portion 33 of the wire 3 on three sides. Importantly, the support 365 makes contact or is close to a side of the third portion 33 that is opposite the first portion 31. When a force F is imparted on the wire (see FIG. 74), the third portion 33 is urged to straighten in a direction G. That straightening force is counteracted with the presence of the vertical second leg 368 acting as a stopping surface. In this embodiment, the wire 3 approaches the tangs 360 from the plate 361 opposite the support 365 and bends around to end inside the support 365.

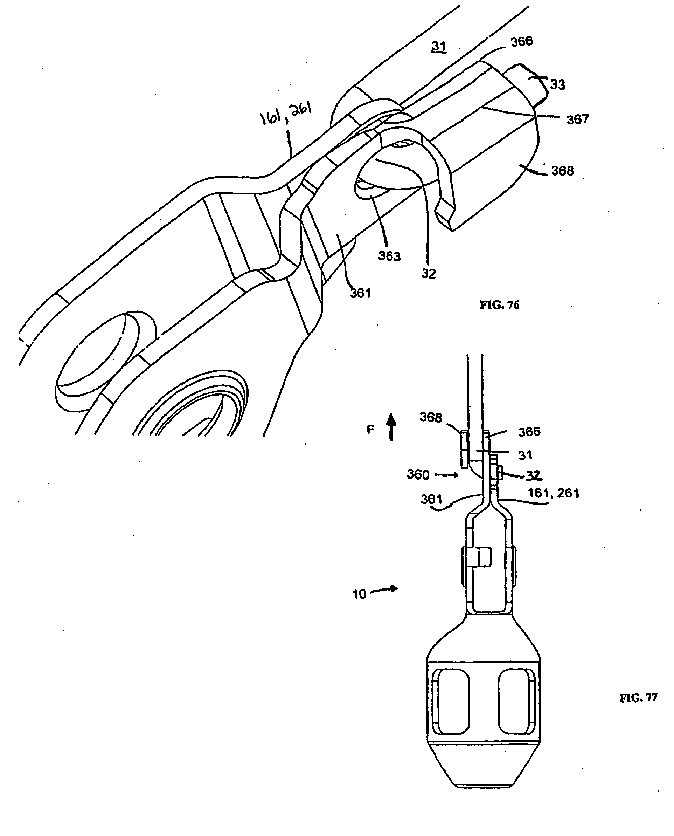

FIGS. 77 to 79 illustrate the third embodiment of the wires 3. Like FIGS. 74 to 76, only one jaw 10 is shown. Each jaw 10 has its respective wire 3 and, therefore, the description herein is not repeated. Unlike FIGS. 74 to 76, in the third embodiment, the wire 3 approaches the tangs 360 through the support 365 and bends around to end inside the tang 360.

To attach the wire 3 to the jaw 10, the wire 3 extends longitudinally from the proximal end of the forceps device up to the control rod port 363 along a first portion 31, bends ninety degrees to enter into the control rod port 363, extends through both the control rod ports 363 of each of the two parallel plates 361 along an orthogonal portion 32, exits the control rod port 363 of the second parallel plate 361, and ends. This third embodiment of the wire 3 similarly reduces any possibility that the wire 3 becomes dislodged from the tang 360.

To effect the reduction of unintended wire 3 removal, one plate 361 of the two parallel plates 161, 261 has a hooded wire support 365. The hooded wire support 365 is configured provide support to the wire 3 and, thereby prevent removal of the wire 3 from the tang 360. The hooded wire support 365 is substantially U-shaped in cross-section and, therefore, has a vertical first leg 366, a horizontal portion 367, and a vertical second leg 368 opposite the first leg 366. The vertical first leg 366 is integral with the plate 361 and extends therefrom at one edge of a proximal portion of the plate 361 (of course it can extend from the other edge or from both edges). The support 365 surrounds the first portion 31 of the wire 3 on three sides. Importantly, the support 365 makes contact or is close to a side of the first portion 31 that is opposite the plate 161, 261. When a force F is imparted on the wire (see FIG. 77), the horizontal portion 32 is urged to straighten. That straightening force is counteracted with the presence of the vertical second leg 368 acting as a stopping surface.

It is noted that the two actuating wires 3 are not illustrated as extending through the entirety of the body 2, 4 and into the handle 62 because such illustration is deemed to be unnecessary. Nonetheless, the preferred embodiment of the present invention provides the two actuating wires 3 with a length that extends all the way into the handle 62 and through most (if not all) of the rod 5 and, in particular, through the three sections 511, 513, 515 of the proximal connecting portion 51 of the rod 5, because it is the bends of the three sections 511, 513, 515 that form a structure that longitudinally holds the two wires 3 within the rod 5.

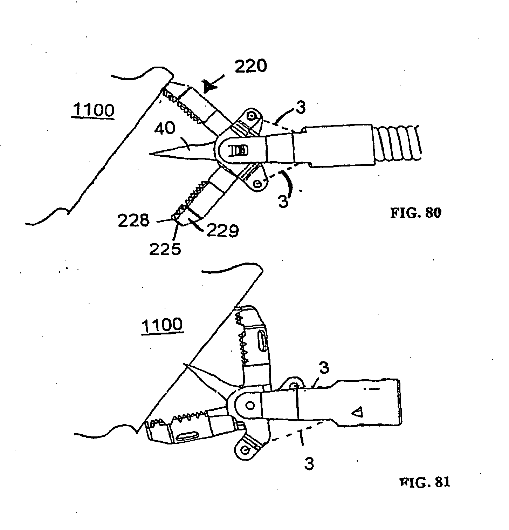

The configuration of the wires 3 are mentioned here to assist in the explanation of a significant feature of the present invention that is particularly illustrated in FIGS. 80 and 81.

When biopsy instruments present themselves to a tissue to be sampled, it is a rare occurrence for the front face of the biopsy jaws to be exactly orthogonal to the tissue to be sampled. This non-orthogonal angle-of-attack means that, when the jaws are pressed into the tissue 1100 to be sampled, the tissue 1100 will not entirely fill up the mouth of the jaws (the rear corner of the mouth portion further away from the tissue will be empty). To insure that there is a maximum volume of tissue sampled, the jaws 220 are formed with the linear front face 225 and angled sides 229. The edge 228 (formed between the front face 225 and each side 229) forms a gripping corner that, when pressed against tissue 1100 to be sampled, plants itself in the tissue 1100 and, naturally, creates a pivoting force that attempts to orient the front face 225 in a direction parallel to the surface of the tissue 1100. This movement of the jaws 220 from an off-axis approach to an orthogonal approach ensures that there is a maximum volume of tissue captured in the mouth portion 221 of the jaws 220 when the jaws 220 are closed.

Prior art biopsy forceps, such as the Radial Jaw^{™} forceps manufactured by Boston Scientific, contained a center spike between the jaws. However, the spike of this prior art forceps remains longitudinally aligned with the clevis (as the orientation of the needle 40 shown in FIG. 80) even if the jaws are pivoted into an orientation shown in FIG. 81. The needle 40 of the present invention, in contrast, pivots with the two jaws 220 to remain centered therebetween when the jaws 220 are fully opened anywhere between the position shown in FIG. 80 and the offset position shown in FIG. 81 (of course the jaws 220 can pivot in the opposite direction than that illustrated in FIG. 81). This pivoting of the needle 40 ensures that the problem of the prior art does not occur -- the needle no longer crushes the biopsy sample that is contained in the close side jaw.

The length of each of the actuating rods 3 inside the coil 2 (which is flexible and, therefore, constructed to bend) are sufficient to compensate for the differential lengths that are needed to place the jaws 220 and the needle 40 in the position illustrated in FIG. 81. Of course, there is the possibility of an alternative embodiment where the two wires 3 are significantly shorter and, instead, connect to a distal end of a third non-illustrated connector that extends through the body 2, 4 to longitudinally connect the rod 5 in the handle 60 to the two wires 3. However, in such an embodiment, if the wires 3 are too short, then there will not be enough play for the wires 3 to permit the offset centering of the end effector 220 against tissue that is presented at an angle to the distal end of the end effector 220 as described above with respect to FIGS. 80 and 81.

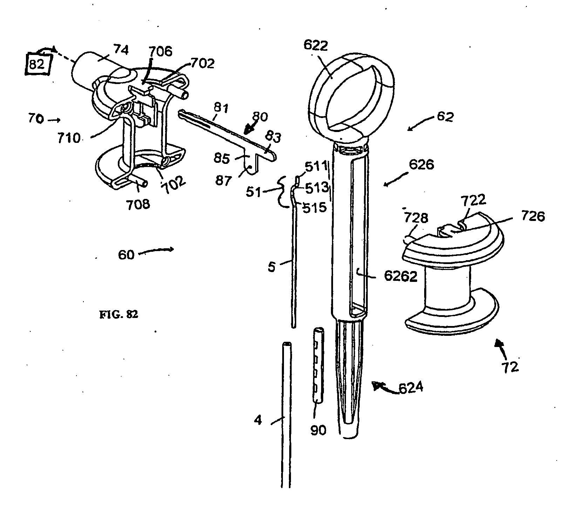

Actuation of the end effector 1 of the present invention occurs by applying a force on the wires 3 as shown by the comparison of FIGS. 28 and 29 or 36 and 37. To close the jaws 10, the wires 3 are drawn in a proximal direction and to open the jaws 10, the wires 3 are extended in a distal direction. The embodiments of the wires 3 illustrated herein all show two separate wires 3 each respectively connected to one of the jaws 10 at a distal end thereof. The proximal end of the wires 3 are not illustrated but are each connected to the distal end of a single actuating rod 5. The proximal end of the actuating rod 5 is illustrated in FIG. 82 with the ellipses indicating that the rod 5 and/or the wires 3 therein extend in the distal direction to the end effector.

FIG. 82 illustrates all of the parts making up the proximal actuator or handle 60 of the present invention. The proximal-most portion of the jacketed coil (including the outer coating 4 and the inner coil 2) is shown separated from the actuating rod 5 (which, in the assembled condition, extends through the coil 2). The actuating rod 5 has, at the proximal end, a connection portion 51 for connecting the rod 5 to a handle center 62 in a manner that will be explained in greater detail below.

The actuating portions of the handle 60 are composed of the handle center 62, which, in the preferred embodiment, has a thumb ring 622 for receiving therein a thumb of a user, and a two-part spool 70, 72 for receiving at least one finger of a user, preferably, at least the index and middle fingers. The first part 70 of the spool contains features for applying cauterizing energy to the metallic jaws 10 and for securing the two spool parts together about the handle center 62. Cauterizing is not a required function of the forceps of the present invention but is preferred when tissue samples being taken will be better obtained when the patient's sample injury area is sealed by cauterization. Therefore, the present invention will be described with a cauterization feature. The second spool part 72 also can contain features for securing the two spool parts together about the handle center 62.

The cautery spool part 70 houses an electrically conducting cautery plug 80 for connecting to a cauterizing energy supply device 82 (illustrated only diagrammatically in FIG. 82). The supply device 82 engages a cautery supply connector (e.g., in the form of a plug receiver 74) disposed on the cautery spool part 70 for supplying cauterizing energy to the cautery plug 80. The cautery plug 80 is made of metal, for electrical conductivity, such as stainless steel but, preferably, copper, or copper alloys such as phosphor bronze, or beryllium copper.

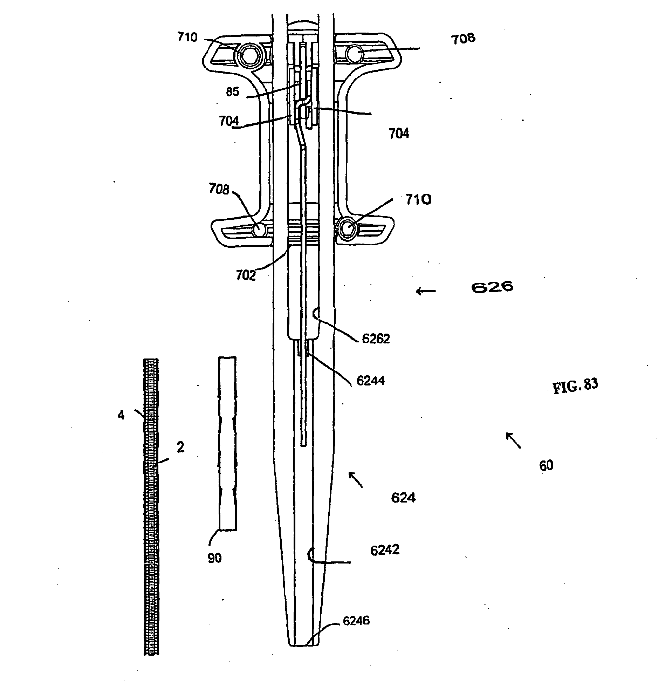

The preferred embodiment of the cautery plug 80 is formed with a supply connector 81 having a shape that can removably connect to the supply device 82. This supply connector 81 extends through a plug receiver 74 that is only illustrated in part in FIG. 82. Preferably, this receiver 74 is shaped to receive a standard cautery supply plug in conventional cautery supply device. In particular, the orifice of the receiver 74 and the supply connector 81 are shaped to removably connect to a cautery supply device manufactured by Olympus and/or Microvasive. A plug orifice is formed in the cautery spool part 70 for receiving the cautery plug 80 in a form-fitting manner and can be seen partially in FIG. 83 by the outline of the cautery plug 80. A form-locking or form-fitting connection is one that connects two elements together due to the shape of the elements themselves, as opposed to a force-locking connection, which locks the elements together by force external to the elements.

On the cautery plug 80, a spool connector 83 is disposed on a side opposite the supply connector 81 and form-fittingly inserts into a non-illustrated orifice located on the interior of the second spool part 72. Thus, when the two spool parts 70, 72 are connected together to surround the handle center 62 (and slide thereon as will be explained below), the cautery plug 80 is held in place.

The cautery plug 80 also needs to contact the rod 5 so that the cauterizing energy can be transmitted from the supply device 82 through the plug 80, then through the rod 5 and wires 3, to the jaws 10 of the end effector 1. To make such a contact, the cautery plug 80 has a rod connection device 85, which, in a preferred embodiment, is in the form of a downwardly extending flange 85 defining a hole 87 for receiving therethrough the proximal connecting portion 51 of the rod 5. To attach the connecting portion 51 of the rod 5 to the flange 85, the proximal end 511 of the rod 5 is inserted into the hole 87 in a position orthogonal to the position shown in FIG. 82. Then, the rod is rotated in a direction towards the longitudinal axis of the flange 85 so that a curved portion having a horizontal section 513 rests within the hole 87. At this point, the extent of the rod 5 is parallel to the longitudinal axis of the flange 85 as shown in particular in FIG. 83. A distal portion 515 of the connecting portion slants towards the longitudinal axis so that when the rod bends away from the distal portion 515, the longitudinal axis of the rod 5 is substantially aligned with the longitudinal axis of the flange 85 as shown in FIG. 83.

The handle center 62 has the thumb ring 622 on a proximal end, a cable receiver 624 on a distal end, and a slide portion 626 disposed between the ring 622 and the cable receiver 624. The slide portion 626 is shaped to receive the two spool parts 70, 72 thereat so that the spool 70, 72 can slide between a distal position and a proximal position. The slide portion 626 is also shaped to receive the cautery plug 80 therein between the two spool parts 70, 72 so that the cautery plug 80 travels with the spool 70, 72 when moved and so that the rod 5 moves with the plug 80 and spool 70, 72.

To better explain the connectivity between the moving and stationary parts of the handle 60, reference is made, now, to FIG. 83. Sliding movement of the spool 70, 72 is created by forming the outer circumference of the handle center 62 in a curved manner, preferably, in a circle. Similarly, inside bearing and sliding surfaces 722, 702 are respectively formed on the interior of the spool part 72 and the cautery spool part 74.

Free movement of the cautery plug 80 (disposed between the spool parts 70, 72) is permitted by forming a longitudinal slot 6262 inside the handle center 62. As such, when the spool 70, 72 moves up and down along the slide portion 626, the cautery plug 80 moves as well. Such movement, therefore, causes the rod 5 to move with and dependent upon movement of the cautery plug 80. So that the connecting portion 51 of the rod 5 does not rotate, swivel, or rock, each of the spool parts 70, 72 contains bearing surfaces. Two of these bearing surfaces 704 are shown in FIG. 83 on the cautery spool part 70.

To prevent the spool 70, 72 from spinning around the longitudinal axis of the handle center 62, each of the spool parts 70, 72 contains a portion of an anti-spinning device 706, 726 in the form of a flange having a distance slightly less than a width of the slot 6262. Of course, one or the other of the spool parts 70, 72 can contain this device 706, 726. However, in the embodiment shown, each spool part contains half of the device 706, 726.

There are many ways to connect the two spool parts 70, 72 together, whether removably or permanently. FIGS. 82 and 83 illustrate one preferred way to connect the spool parts 70, 72 together. Each spool part 70, 72 contains two pegs 708, 728 and two connector receiving ports 710, 720. The pegs 708, 728 can be press fit into the ports 710, 720 in a form-fitting and/or a force-fitting manner. In the embodiment shown in FIGS. 82 and 83, the pegs 708, 728 are hollow and can have a smooth or threaded interior. Non-illustrated screws or bolts are inserted through the ports 710, 720 from a side opposite the pegs 708, 728 and screw or thread into the interior of the pegs 708, 728.

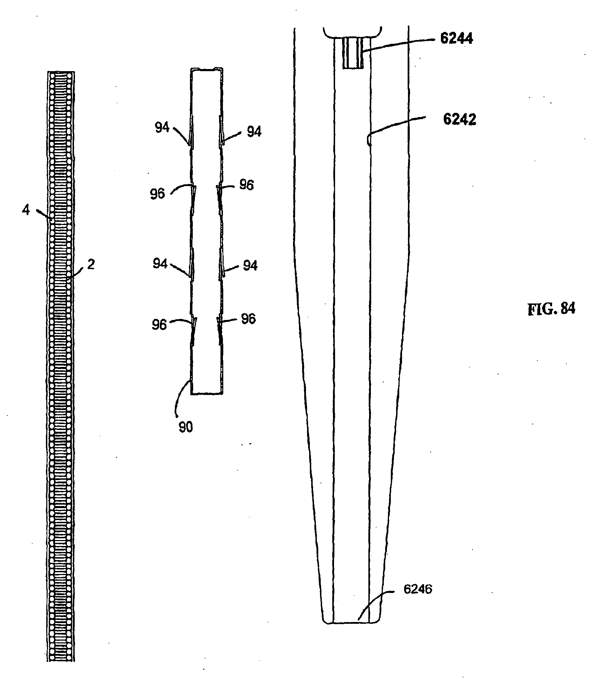

Connection of the shaft 2, 4 to the handle center 62 occurs at the cable receiver 624 as shown in FIGS. 83 to 85. The cable receiver 624 has an interior bore 6242 from its proximal end to its distal end. The bore 6242 has a constant diameter. However, an integral spacer 6244 is disposed inside the proximal end of the bore 6242 in a co-axial orientation. The spacer 6244 functions in two ways. First, it centers the rod 5 after the rod 5 has been threaded through the bore 6242 from the distal end through the proximal end to, thereafter, be threaded through the hole 87 of the flange 85 of the cautery plug 80. The spacer 6244 is shallow enough to not prevent the bent connecting portion 51 from passing therethrough. Second, the spacer 6244 acts as a proximal stop for the shaft 2, 4 to prevent further proximal threading of the shaft 2, 4 through the bore 6242.

However, if the shaft 2, 4 is merely inserted into the bore 6242, it will not be held therein. It is desirable to have a single component that accomplishes the task of connecting the shaft 2, 4 to the handle center 62 without the use of adhesives and without any additional crimping or forming operations. Accordingly, a shaft retainer 90 is provided.

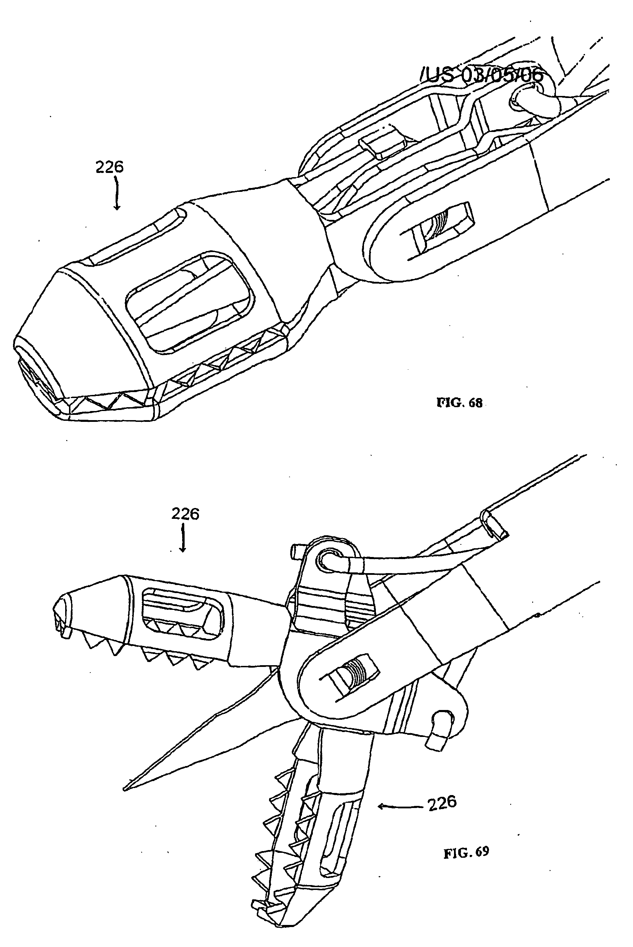

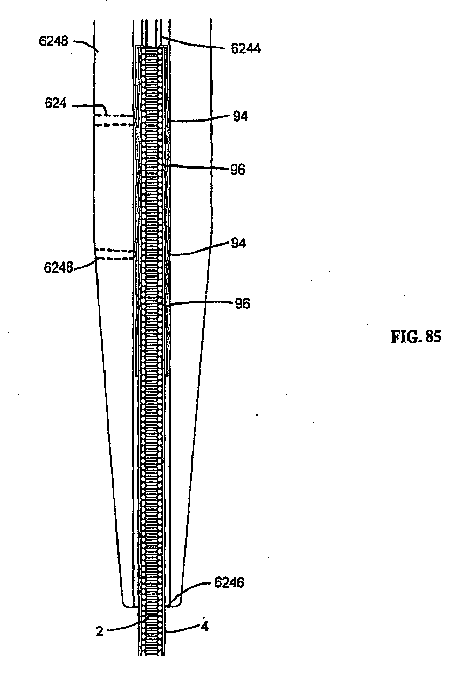

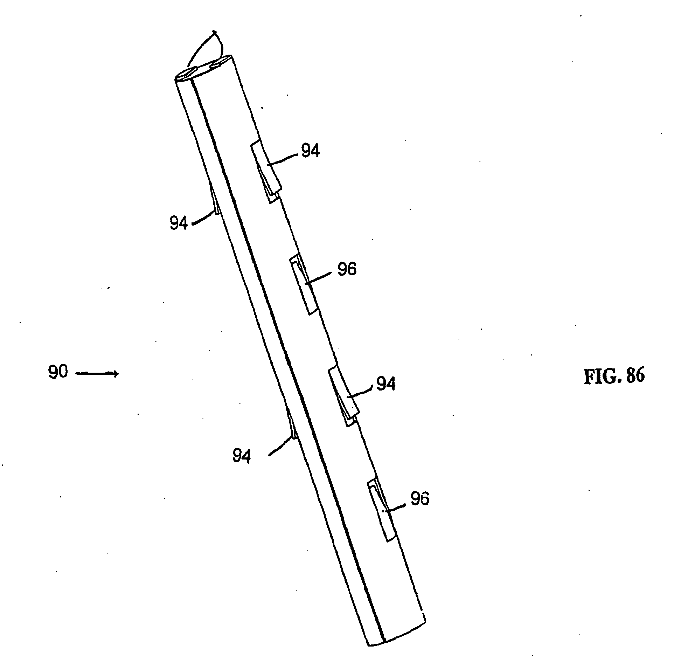

The shaft retainer 90 is a device that grasps onto both the cable receiver 624 and the polymer coated material 4 around the spring coil shaft 2 to join the two parts together. The shaft retainer 90 simply slides over the proximal end of the shaft 2, 4. To control the depth of insertion, in a first embodiment shown in FIG. 86, the shaft retainer 90 has a stop 92 formed by folded over tabs. In a second embodiment shown in FIG. 68, the stop 92 can be formed with dimples, coffee spouts, or any other shape that protrudes into the internal diameter of the shaft retainer 90 sufficient to stop entry of the shaft 2, 4. The shaft 2, 4, with the retainer 90 attached, is, then, inserted into a hole 6246 at the distal end of the handle until it hits the spacer 6244. This fully inserted and connected position is illustrated in FIG. 85 (the rod 5 is removed for clarity).

Merely providing the shaft retainer 90 on the proximal end of shaft 2, 4 may not be sufficient to prevent removal of the shaft 2, 4 if a distal force is exerted upon the shaft 2, 4. Therefore, the shaft retainer 90 is formed with outwardly projecting tines 94 and inwardly projecting tines 96. The outwardly projecting tines 94 are flattened as the shaft retainer 90 is pressed into the bore 6242. But, when such movement stops, the outward bias of the tines 94 press against the interior wall of the bore 6242. The sharpness and hardness of the tines 94 combined with the relative softness of the interior of the bore 6242 (which is, preferably, a plastic) digs into the bore 6242 and prevents withdrawal of the shaft retainer 90 from the bore 6242. If the bore 6242 is provided with interior roughness (whether random, threaded, or periodic), then the tines 94 may be assisted in firmly contacting the interior wall of the bore 6242 by providing spaces for the tines 94 to spring. In such a configuration, the shaft retainer 90 is locked in the bore 6242. The locking can also be accomplished with holes 6248 that penetrate through the body of the cable receiver 624 (illustrated only diagrammatically with dashed lines in FIG. 85.

The above connection securely connects the shaft retainer 90 to the cable receiver 624. The shaft retainer 90 is further provided with inwardly projecting tines 96. These tines 96 protrude toward the inside of the shaft retainer and, after the shaft 2, 4, is inserted therein, dig into the softer plastic coating of the protective material 4 around the spring coil 2 and may even penetrate enough to grab onto the ridges of the coil 2 itself as shown in FIG. 85. Thus, without the use of adhesives and without any additional crimping or forming operations, the shaft retainer 90 fixedly connects the shaft 2, 4 to the handle center 62 in a very simple manufacturing step.

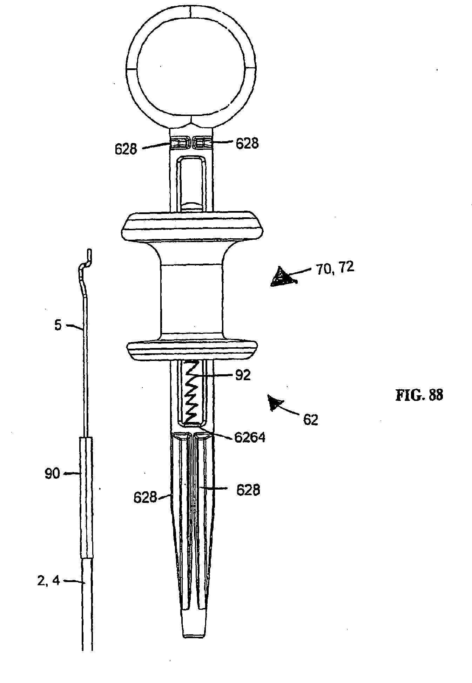

FIG. 88 illustrates the assembly containing the rod 5, the shaft 4, 2, and the shaft retainer 90 separate from the assembly containing the handle center 62 and the spool 70, 72 holding the cautery plug 80. To conserve material when manufacturing the handle center 62, cutouts 628 may be provided in any manner or shape on the exterior of the handle center 62.

Because a forceps user desires to capture a sample within the closed jaws 10, it is desirable to have the end effector 1 biased in a closed position. Thus, a bias device can be provided between the handle center 62 and the spool 70, 72. For example, a spring 92 can be disposed inside the longitudinal slot 6262 and around the rod 5. In such a configuration, the spool 70, 72 can be provided with a surface similar to the anti-spinning device 706 but on the lower set of bearing and sliding surfaces 722, 702 or the anti-spinning device 706 can be disposed on the lower side of the spool 70, 72 and, thereby, provide the upper bearing surface of the spring 92. The opposed lower bearing surface of the spring 92 is provided by the lower surface 6264 of the longitudinal slot 6262 (see FIG. 88).

As set forth herein, it would be beneficial to improve the prior art indicator marks that have been added to the distal portion of the sheath to give the physician a visual cue that the tip of the instrument is approaching, for example. Improvement of printing such marks was also discussed and it was observed that it would be desirable to eliminate any possibility of damaging the delicate outer sheath by adding better indicating markers.

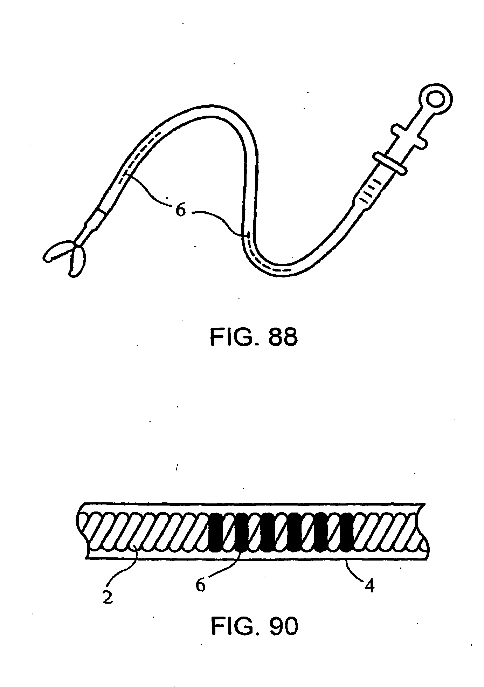

FIGS. 89 and 90 diagrammatically illustrate a medical instrument according to the present invention having an end effector 1 (e.g., a forceps 10, 20, 30) and a handle 60 connected to the end effector 1 through a shaft. The shaft includes an interior coil 2 and an outer sheath 4 covering the coil 2.

Indicating markers 6 for the, in particular, endoscopic instrument are applied to the coil 2 of the shaft and are covered by a clear sheath 4. As indicated above, prior art outer sheaths are opaque, and color coded to indicate overall length (orange=240 cm, yellow=160 cm, blue=100 cm for instance). The markers of the present embodiment, 6 can be colored to indicate the length of the device. The sheathing operation, therefore, is not unique to the length of the device and, in such a configuration, streamlines production. All sizes are covered by the same clear sheath material and can be run on one extrusion line without having to change materials. Also, because the markers 6 are applied directly to the coil 2, there is no chance for the delicate polymer of the outer sheath 4 to be damaged during printing. Because the markers 6 are covered by the outer sheath 4, they can include inks that might not be useable otherwise on the outside surface of such a medical device, e.g., glow-in-the-dark inks. Additionally, markers 6 according to the present invention are more durable than the printed exterior marks of the prior art.

The markers 6 are applied, in particular, to the coil 2 of the shaft during manufacture while the coil 2 is being wound and cut to length. The markers 6 can be of a specific color corresponding industry standards. The markers 6 can correspond to length of the shaft 2, 4 from the end effector 1 or correspond to the length of the shaft 2, 4, from the handle 60. In the former embodiment, for example, a red band can indicate 5 cm from the end effector 1, orange can indicate 10 cm therefrom, yellow can indicate 15 cm therefrom, green can indicate 20 cm therefrom, blue can indicate 25 cm therefrom, and violet can indicate 30 cm therefrom. Alternatively, or additionally, a non-illustrated scale, such as a metric ruler, can be added to the length of the coil 2 and, then, covered by the transparent outer sheath 4.

One method of creating such a coil 2 with markers 6 can be implemented as the coil 2 is wound on a continuous coil winder. One end of the spinning coil 2, as it increases in length from the beginning of winding, passes through an optical sensor. This sensor can be configured to trigger a color spray head or a print head or a pad print head to mark the coil 2 until the end of the spinning coil 2 trips another sensor that stops the printing. The so-labeled coil 2, then, passes through a forced air and/or high temperature curing chamber as it is further wound. The coil 2 is, then, cut to length for discrete length overextrusion, or taken up on a spool in a long continuous length for subsequent continuous overextrusion. In the case of a continuous long length of coil for subsequent overextrusion, the printing sensors on the coiling machine can be controlled either by time or by the revolutions of the rollers feeding the wire into the coiling machine. In this way, the marks are printed in the correct locations on the coil.

The markers 6 can also be thin rings of material such as those shown in the partially cut-away view of FIG. 90.

Use of the jaws and clevis has been explained in the description of the present invention for a biopsy forceps. It is to be noted, however, that the present invention is not so limited. The device and method according to the invention can be used with any need. For example, the same tang configuration could be used for many different kinds of end effectors such as: graspers, dissectors, clamps, etc. in many areas of surgery, such as laparoscopic, general, arthroscopic, etc., both for rigid and flexible instruments.

## Claims

1. An end effector for an endoscopic surgical instrument, comprising:
a pair of jaws each comprising:
a tang portion (240);
a hollow body portion (221) having:
a lateral side (223) with substantially straight opposing edges; and
a proximal portion (227) connecting said lateral side (223) to said tang portion (240); and
a hollow nose portion (225, 229) connected to said lateral side (223) and having substantially linear edges (229) at an angle to said edges of said lateral side (223), said hollow body portion (221) and said hollow nose portion (225, 229) defining a biopsy cup for receiving a tissue sample therein, said cup having a perimeter, said opposing edges and said linear edges (229) meeting at respective corners each defining an outer surface transition discontinuity; and
said perimeters of said cups being substantially equal in shape and aligned to form a pinching surface for contacting extremities of the tissue sample.

2. The end effector according to claim 1, wherein said edges of said nose portion (225, 229) are each connected at an angle to a respective one of said opposing edges (223) of said lateral side (223).

3. The end effector according to claim 2, wherein :
said linear edges (229) of said nose portion (225, 229) are two linear edges;
said opposing edges (223) of said lateral side (223) are two opposing edges; and
said two edges (225, 229) of said nose portion (225, 229) are each connected at an angle to a respective one of said two opposing edges (225, 229).

4. The end effector according to claim 1, wherein said linear edges (229) of said nose portion (229) are two linear edges.

5. The end effector according to claim 4, wherein said two linear edges (229) oppose one another.

6. The end effector according to claim 5, wherein said two linear edges (229) oppose one another at an angle.

7. The end effector according to claim 1, wherein said nose portion (225, 229) forms one half of a frustoconical body having two substantially linear side edges (229), a substantially linear front edge (225), and a substantially linear front face (225).

8. The end effector according to claim 1, wherein said nose portion has:
a side portion (229) connected to said lateral side (223)and having said linear edges (229) at said angle to said lateral side (223); and
a front portion (225, 229) having:
a substantially linear front face (225); and
a linear edge (225) connected at an angle to each of said edges (229) of said side portion.

9. The end effector according to claim 8, wherein said side portion (223) and said front portion (225, 229) form one half of a frustoconical body.

10. The end effector according to claim 8, wherein said side portion (229) is a set of angled side portions.

11. The end effector according to claim 10, wherein said set of angled side portions (229) forms one half of a bi-frustoconical body.

12. The end effector according to claim 1, wherein at least one of said opposing edges (223) and said linear edges (229) has at least one tooth.

13. The end effector according to claim 1, wherein said opposing edges (223) and said linear edges (229) each has at least one tooth.

14. The end effector according to claim 1, wherein said opposing edges (223) and said linear edges (229) each have teeth.

15. The end effector according to claim 8, wherein said linear edge (225) of said front portion has at least one tooth.

16. The end effector according to claim 1, wherein said tang portion (240), said body portion, and said nose portion (225) are integral.

17. The end effector according to claim 16, wherein said tang potion (240), said body portion (223), and said nose portion (225, 229) are stamped from a single piece of material.

## Patentansprüche

1. Endeffektor für ein endoskopisch-chirurgisches Instrument, mit:
einem Paar von Backen, wobei jede aufweist:
einen Angelbereich (240);
einen Hohlkörperbereich (221) mit:
einer lateralen Seite (223) mit im Wesentlichen geraden gegenüberliegenden Kanten; und
einem proximalen Bereich (227), welcher die laterale Seite (223) mit dem Angelbereich (240) verbindet; und
einen hohlen Nasenbereich (225, 229), welcher mit der lateralen Seite (223) verbunden ist und im Wesentlichen lineare Kanten (229) in einem Winkel zu den Kanten der lateralen Seite (223) aufweist, wobei der Hohlkörperbereich (221) und der hohle Nasenbereich (225, 229) eine Biopsieschale zum Aufnehmen einer Gewebeprobe darin definieren, wobei die Schale einen Durchmesser besitzt, wobei die gegenüberliegenden Kanten und die linearen Kanten (229) sich an jeweiligen Ecken treffen, die jeweils eine äußere Oberflächenübergangsunstetigkeit definieren;
wobei die Durchmesser der Schalen im Wesentlichen gleich sind in Form sowie so angeordnet sind, dass sie eine klemmende Fläche bilden zum Kontaktieren von Rändern der Gewebeprobe.

2. Endeffektor nach Anspruch 1,
wobei die Kanten des Nasenbereichs (225, 229) jeweils in einem Winkel mit einer jeweiligen der gegenüberliegenden Kanten (223) der lateralen Seite (223) verbunden sind.

3. Endeffektor nach Anspruch 2, wobei:
die linearen Kanten (229) des Nasenbereichs (225, 229) zwei lineare Kanten sind;
wobei die gegenüberliegenden Kanten (223) der lateralen Seite (223) zwei gegenüberliegende Kanten sind, und
wobei die zwei Kanten (225, 229) des Nasenbereichs (225, 229) jeweils in einem Winkel mit einer jeweiligen der zwei gegenüberliegenden Kanten (225, 229) verbunden sind.

4. Endeffektor nach Anspruch 1,
wobei die linearen Kanten (229) des Nasenbereichs (229) zwei lineare Kanten sind.

5. Endeffektor nach Anspruch 4,
wobei die zwei linearen Kanten (229) einander gegenüberstehen.

6. Endeffektor nach Anspruch 5,
wobei die zwei linearen Kanten (229) einander in einem Winkel gegenüberstehen.

7. Endeffektor nach Anspruch 1,
wobei der Nasenbereich (225, 229) eine Hälfte eines kegelstumpfförmigen Körpers mit zwei im Wesentlichen linearen Seitenkanten (229), einer im Wesentlichen linearen Vorderkante (225) und einer im Wesentlichen linearen Vorderfläche (225) bildet.

8. Endeffektor nach Anspruch 1, wobei der Nasenbereich aufweist:
einen Seitenbereich (229), welcher mit der lateralen Seite (223) verbunden ist und welcher die linearen Kanten (229) in dem Winkel zur lateralen Seite (223) aufweist; und
einen Vorderbereich (225, 229) mit:
einer im Wesentlichen linearen Vorderfläche (225); und
einer linearen Kante (225), welche in einem Winkel mit jeder der Kanten (229) des Seitenbereichs verbunden ist.

9. Endeffektor nach Anspruch 8,
wobei der Seitenbereich (223) und der Vorderbereich (225, 229) eine Hälfte eines kegelstumpfförmigen Körpers bilden.

10. Endeffektor nach Anspruch 8,
wobei der Seitenbereich (229) eine Gruppe gewinkelter Seitenbereiche ist.

11. Endeffektor nach Anspruch 10,
wobei die Gruppe gewinkelter Seitenbereiche (229) eine Hälfte eines doppelt kegelstumpfförmigen Körpers bildet.

12. Endeffektor nach Anspruch 1,
wobei mindestens eine der gegenüberliegenden Kanten (223) und der linearen Kanten (229) mindestens einen Zahn aufweist.

13. Endeffektor nach Anspruch 1,
wobei die gegenüberliegenden Kanten (223) und die linearen Kanten (229) jeweils mindestens einen Zahn aufweisen.

14. Endeffektor nach Anspruch 1,
wobei die gegenüberliegenden Kanten (223) und die linearen Kanten (229) jeweils Zähne aufweisen.

15. Endeffektor nach Anspruch 8,
wobei die lineare Kante (225) des Vorderbereichs mindestens einen Zahn aufweist.

16. Endeffektor nach Anspruch 1,
wobei der Angelbereich (240), der Körperbereich und der Nasenbereich (225) einstückig sind.

17. Endeffektor nach Anspruch 16,
wobei der Angelbereich (240), der Körperbereich (223) und der Nasenbereich (225, 229) aus einem einzelnen Materialstück gestanzt sind.

## Revendications

1. Organe effecteur terminal pour un instrument chirurgical endoscopique comprenant :
une paire de mâchoires comprenant chacune :
une partie tenon (240) ;
une partie corps creux (221) comportant :
un côté latéral (223) comportant des bords opposés sensiblement droits ; et
une partie proximale (227) reliant ledit côté latéral (223) à ladite partie tenon (240) ; et
une partie frontale creuse (225, 229) reliée à ladite partie latérale (223) et comportant des bords sensiblement rectilignes (229) faisant un certain angle avec lesdits bords dudit côté latéral (223), ladite partie corps creux (221) et ladite partie frontale creuse (225, 229) définissant une coupelle à biopsie pour recevoir un échantillon de tissu, ladite coupelle ayant un certain périmètre, lesdits bords opposés et lesdits bords rectilignes (229) se rencontrant au niveau d'angles respectifs définissant chacun une discontinuité de transition de surface externe ; et
lesdits périmètres desdites coupelles étant sensiblement égaux sur le plan de la forme et alignés afin de former une surface de pincement pour mettre en contact les extrémités de l'échantillon de tissu.

2. Organe effecteur terminal conformément à la revendication 1, dans lequel lesdits bords de ladite partie frontale (225, 229) sont chacun reliés, selon un certain angle, à un bord respectif parmi les bords opposés (223) dudit côté latéral (223).

3. Organe effecteur terminal conformément à la revendication 2, dans lequel :
lesdits bords rectilignes (229) de ladite partie frontale (225, 229) sont deux bords rectilignes ;
lesdits bords opposés (223) dudit bord latéral (223) sont deux bords opposés ; et
lesdits deux bords (225, 229) de ladite partie frontale (225, 229) sont chacun reliés, selon un certain angle, à un bord respectif parmi lesdits deux bords opposés (225, 229).

4. Organe effecteur terminal conformément à la revendication 1, dans lequel lesdits bords rectilignes (229) de ladite partie frontale (229) sont deux bords rectilignes.

5. Organe effecteur terminal conformément à la revendication 4, dans lequel lesdits deux bords rectilignes (229) se font face mutuellement.

6. Organe effecteur terminal conformément à la revendication 5, dans lequel lesdits deux bords rectilignes (229) se font face mutuellement selon un certain angle.

7. Organe effecteur terminal conformément à la revendication 1, dans lequel ladite partie frontale (225, 229) forme une moitié d'un corps tronconique ayant deux bords latéraux sensiblement linéaires (229), un bord avant sensiblement linéaire (225) et une face avant sensiblement linéaire (225).

8. Organe effecteur terminal conformément à la revendication 1, dans lequel ladite partie frontale comprend :
une partie latérale (229) reliée audit côté latéral (223) ayant lesdits bords rectilignes (229) selon ledit angle par rapport audit côté latéral (223) ; et
une partie avant (225, 229) ayant :
une face avant sensiblement rectiligne (225) ; et
un bord rectiligne (225) relié, selon un certain angle, à chacun desdits bords (229) de ladite partie latérale.

9. Organe effecteur terminal conformément à la revendication 8, dans lequel ladite partie latérale (223) et ladite partie avant (225, 229) forment une moitié d'un corps tronconique.

10. Organe effecteur terminal conformément à la revendication 8, dans lequel ladite partie latérale (229) est un ensemble de parties latérales angulaires.

11. Organe effecteur terminal conformément à la revendication 10, dans lequel ledit ensemble de parties latérales angulaires (229) forme une moitié d'un corps bitronconique.

12. Organe effecteur terminal conformément à la revendication 1, dans lequel lesdits bords opposés (223) et/ou lesdits bords rectilignes (229) comportent au moins une dent.

13. Organe effecteur terminal conformément à la revendication 1, dans lequel lesdits bords opposés (223) et lesdits bords rectilignes (229) comportent chacun au moins une dent.

14. Organe effecteur terminal conformément à la revendication 1, dans lequel lesdits bords opposés (223) et lesdits bords rectilignes (229) comportent chacun des dents.

15. Organe effecteur terminal conformément à la revendication 8, dans lequel ledit bord rectiligne (225) de ladite partie avant comporte au moins une dent.

16. Organe effecteur terminal conformément à la revendication 1, dans lequel ladite partie tenon (240), ladite partie corps, et ladite partie frontale (225) sont d'un seul tenant.

17. Organe effecteur terminal conformément à la revendication 16, dans lequel ladite partie tenon (240), ladite partie corps (223) et ladite partie frontale (225, 229) sont estampées dans une pièce de matériau unique.
